# EUROPEAN PATENT APPLICATION

(11) **EP 3 933 029 A2**
(43) Date of publication of application: **05.01.2022**
(21) Application number: 21169279.3
(22) Date of filing: 29.09.2009
(51) Int. Cl.: C12N 1/00

(54) **REDUCED COLONIZATION OF MICROBES AT THE MUCOSA**

(30) Priority: 29.09.2008 EP 08105458
(62) Divisional of application: 17154984.3
(71) Applicant: Intrexon Actobiotics NV, 9052 Zwijnaarde (BE)
(72) Inventor: STEIDLER, Lothar, 9160 Lokeren (BE)
(74) Representative: Pohlman, Sandra M.

(57) **Abstract**

The invention is in the field of use of engineered microbes for the delivery and administration of therapeutic peptides or proteins to humans or animals suffering from a disease, or the use of engineered microbes for the delivery of antigens such as for vaccination purposes. More in particular, the invention relates to a recombinant microbe that has reduced capacity of colonizing the mucosa in comparison to its wild type ancestor, in particular when residing in the alimentary tract as part of a treatment or vaccination of a human or animal. In particular, the recombinant microbe contains an inactive thymidylate synthase gene that causes the reduced capability for the microbe to colonize in the alimentary tract. The invention also covers the use of said recombinant microbes comprising nucleic acids or vectors for expressing heterologous or homologous proteins; and also for delivery, especially therapeutic delivery, of the said proteins to animals or humans.

SEQUENCE LISTING

<110>  Intrexon Actobiotics N.V.

<120>  REDUCED COLONIZATION OF MICROBES AT THE MUCOSA

<130>  INX16605PCTEPD2

<140>  Not yet assigned
<141>  2009-09-29

<150>  EP 17154984.3
<151>  2009-09-29

<150>  EP 09736880.7
<151>  2009-09-29

<150>  PCT/EP2009/062647
<151>  2009-09-29

<150>  EP 08105458.7
<151>  2008-09-29

<160>  9

<170>  PatentIn version 3.3

<210>  1
<211>  1000
<212>  DNA
<213>  Streptococcus mutans UA 159

<400>  1
caaaattcac cttaagtttt tcctgcaaat cagatgggac ttgcttatcc atgattttgt      60

taaagccttt aagagctagt ttaggaaaag gaagatgaaa gcaaaaagct gcaaaatcag     120

ttaaagaaac atcaaaacgt ttttgatatt ctgcccaagt cgttttcagc atatccaaat     180

attgtttggt agaatacata ccattaacat aaggtgttgt tgtataattt ggccgccaaa     240

aatccataat atcacgtgtc tgagctaagg tctcatcatg aagaataaga atacggggat     300

cttttttgac cagcatggca atgctgccgg ctccttgagt agattctccg ggagttccaa     360

ttccgtattt ggcaatatcg ctagcaagaa caagcacacg cgtgtcagga tgtttttcaa     420

catgcagttt agcatagttg agagctgcag tagcactgta gcatgcctct ttcatctcaa     480

agctgcgcgc aaaaggctga atacctaata aggaatgcac ataaacagct ccagccttac     540

tttgatcaac gctggattcg gtagctaaaa tgaccatatc aattttttct ttatcctctg     600

cagtcagaat ttcattggct gatccggctg ctagggtgac aacgtcgtcg gtaataggag     660

cgatgctaag agcattcagc aaaagtcctt tactgaattt ttggggatct tcgccacggg     720

cctcagctaa atctttcata ttcaggacat attggctgct tgtaaaacca attttatcaa     780

tcccaattct cataagtcaa tccttctttt cttttttatc tgatttcttt tttgtaaaat     840

gtcatagtta ttttatcaca atttaaacta aaggtaatga ataaactttc aaggattcaa     900

tttttgggag aatctgtcag atcagacgaa acctattttt tctgctataa tatctaaaag 960

aagtttaaat tattttctta gaatcataga aggagaagca 1000

<210> 2
<211> 1000
<212> DNA
<213> Lactobacillus acidophilus NCFM

<400> 2
attttctttg gtgtaggcaa cggtggaaag ccaattggtt ttagcaattt atgggctcat 60

ggcggtttct ttactggcgg agtaaaaggc tttttcttct cgatgtcaat tattgttggt 120

tcatatgaag gaattgagtt gctaggcatt tcggcaggcg aggtggctaa cccgcaaaaa 180

gcaattgtaa agagtgtcaa gtctgttctt ttgagaattt taattttcta cgttggtgca 240

atttttgtga ttgtaactat ttatccgtgg aataagttaa gtagcttagg ttctcctttt 300

gtaactacct ttgctaaggt aggaatcact gctgctgctt cgattattaa cttcgttgta 360

ttaactgcag cattgtcagg agctaactcg gggatttatt catcaagtag aatgctattt 420

aaattgtctc atgataatga agcacctagt gtatttaagc atatttctaa gagaattgtg 480

cctgatcgcg ccattatggg gatttctggt gggattttta ttgggtttat tttaaatata 540

attgcatctc aatttaatca ttcggcatca gatttatttg tgattgtctt cagttcatca 600

gttttaccag gaatgattcc atggtttgta attctattgg ctgagttaag atttagaaga 660

cataatcaag atatgatgaa agatcacccg ttcaaattgc cgttatatcc attttctaat 720

tacttcgcat ttttaatgct gttagtaatt gttatcttta tgtttattaa tccagatact 780

agaatttcag taattaccgg agcattggta ttaattgtgg ctacaattgt ttatttagtt 840

agacataaag atgaatttag taaaaataat taatgattaa gaagtcttga aatttcaggg 900

ctttttattt ttaccaaata ctaaatattg atacttgcat tatcaaaaat actagatcta 960

tggtatctta aaaagaaatg atacttaaag ggtgagataa 1000

<210> 3
<211> 1000
<212> DNA
<213> Lactobacillus plantarum WCFS1

<400> 3
ttcgctcggt cagcagcgtt tgggtaagaa agtgatcgag ttaaaggacg cgagcttgca 60

gtttgatcgg caaaccatcc tggatcactt ttcgatgttg atccaagcca atgaccgcat 120

cggtatcact ggaatcaacg gtgccgggaa atctagttta ttgaatgtga ttgcgggtcg 180

actgccactt gatagtggga ccgtgacgat cggtgagacc gtcaagatgg cctattatac 240

tcaacagacc gaaccgattc cgggcgacaa gcgcatcatt aattacttgc aagatgtcgg 300

tgagacggtc ttgaataagc agggcgagca tgtttcagtg actgagttac tagaagagtt 360

tctgttccca cgttcgatgc acggcacgct gattcgcaag ttatctgggg gcgaacagcg 420

ccggctatac ttactgaagt tgttgatgca acagccgaac gtcctcttat tggacgaacc 480

gactaatgat ttggatattg gtacgttaac ggtgttggag aactacctcg atgattttgc 540

cgggaccgtc atcactgttt cccatgaccg ctattttttg gacaaggtcg gcacgaaact 600

actgattttt gatggtcagg ggcatattga acgctattct ggccgtttct ccagttattt 660

gaaggatcaa aaagacgcgg ccaagcccgc agccaaggcc actgcgacaa aaactacggc 720

taagccggtc acggatgact cgaccgcacc attggccaag aaagccaaag tcaagctgac 780

ttacgcggaa cagctcgaat acgacaagat cgaaggggtt atcgagcaat tggatagcca 840

taagagtgag atcgaagcgg cgatggctgc caatgccagt gattatggca agttggctga 900

tttgcaaaaa gaattgacta agacggaaca aacgattgat gagaagatgg atcgctggga 960

ctacctcagc cagtatgcag aagcctgaaa ggatgatcgc 1000

<210> 4
<211> 1000
<212> DNA
<213> Lactobacillus salivarius UCC118

<400> 4
ttgatattaa tttaggacaa caaagattgg gaaagaaagt attagaatta aaagatgcaa 60

gtttaacaat tggaaatcat aaaataattg aagatttcaa tttgctaatc caagctggag 120

acagaatcgg tattacaggc gttaatggtg ccgggaaatc tagtttttta aatgctttat 180

caggtgaatt gccattagat tcaggtattt tgacaatcgg tgaaactgta aagatggctt 240

attatcgaca acagacggaa gagattcctg aagataaaag aattattagt tatttaaacg 300

aagttggtca aaatattgta aataaagatg gtgagcgaat tagcacaact caattattgg 360

aacaatttct atttccaaga tttatgcatg gaacattgat tcgcaaactc tctggcggtg 420

aaaaaagacg cttgtatctt ttgaaattat taatgtctca acctaatgtt ttattattgg 480

atgaaccaac taacgattta gatataggaa cattaacagt tttggaagat tatttagata 540

actttaacgg aactgttatt accgtatcac atgacagata ttttcttgat aaggtagctg 600

attcattata tatctttgaa ggaaatgcaa aaatcaagca ttatgtcgga atgtttacag 660

attatctgaa gaatgctgag aatgaagcag ttaagactaa aaaacaagta tctgcaacaa 720

aagtagaaaa gacagatagt gcagaagaca aagttaaaaa gaaaaccaag ttaacttacg 780

ctgaaaagat ggaatatgaa aaattagaat cagaaatcga taagcttgaa aatgataaag 840

caagtttaga agaagaaatg caacatgttg atggggctga ttatactaaa ttagcaagtt 900

tacagcaaca aattgatgaa ttagatgaag atattatgga aaaagttcaa cgttgggacg 960

aacttagtca gtatgttgat taataggaag gagaacaccg 1000

<210>  5
<211>  1000
<212>  DNA
<213>  Streptococcus mutans UA 159

<400>  5
aaatgacgag ccaataatta tggctctttt ttataaaaaa ccaatatatg tcttgacaca      60

tgtaaaaaac gttgtataat gatttcaaac attaattaaa aaggtggagc aaaacggtgt     120

ctagaaaagc aagcaatatc tgggataatt ttaaatggct gtggcaagaa ggtaaaaaat     180

taggcttttg ggggatatta caagcgctat gggaggattt ggttaaaaat aggagcctgt     240

cgcagtggct atacctttta gctcttagtt ttccaacact ggttttagaa tttatcggtg     300

gaacacggca cattgctggt tttgcggcag cactgacagg aattctatgt gttatctttg     360

ttgctgaggg gcgtatcagt aattatttca tcggttttat tcatgaaatg ctctatcttt     420

atctcagttt tgaaaatatg tattatggtg aagttttaac aaccttattt ttcactgtca     480

tgcaatttgt cggtgcttat tattggttga ttggacatcg tgaaggacag ggaaagaaag     540

ttgaagtaaa ggatgttaaa tcacgaaaac tgacacccct aggctggttg aaatcacttg     600

gaattacaat tattgtttgg ttagtttttg gctttattta tcgatcaatc ggttcgcatc     660

gccctttttg ggatagttca acagatggta ccaattggag cggtcagttt cttcaaacag     720

gcatgtacag tgaacaatgg ctcttttgga ttgcgaccaa tgttctcagc atttttcttt     780

ggtggggtgc agaaccacat gttatgctca tgtatattat ttatatgatt aatagtattg     840

ttggttgggt aaaatgggaa cgtgatctta gaataactca agaacagtta gcttaaataa     900

ggagtttaaa tacaatagac aaaatgcatt tgagtttttt caatgcattt tttttagaaa     960

tttgataaaa taagaaaagt atgaacaata agcgagaaaa                          1000

<210>  6
<211>  1000
<212>  DNA
<213>  Lactobacillus acidophilus NCFM

<400>  6
ggtgaagtaa tgattgaata tgtttgggca gaagataaag aaaaaaatat tggcttgaat      60

ggacatttac catggtattt gccggctgat atgaagcatt ttaaagaagt aacaattaat     120

catccaataa ttatgggaag aaaaacattt gaaagttttc ctaatttgtt acctaaaaga     180

aaacatattg ttttaactca taatgaagag ctaaaaaata aatatcaaaa taatgatcaa     240

gtgactattt tacccacagt tgaagattta cataattttg tggcagaaca tcaagatgag     300

cggatgtgtg caattggtgg agtgtcgatt tttaacgctt taatggacca agtagaagta     360

ttagaaaaaa cggagataga tgcgattttt gaagcagata ctaaaatgcc tgaaattgat     420

tatagccgtt ttaatttagt agctaagaag cattatgagc cggatgagaa aaataagtat 480

ccatatactt ttttaactta taaattaaag taaaaaaagc ccgcaatttg cgagcttttt 540

tgaatcttag gtataatatt aataaatatt aagtttctta ggtaataata tatgaatgaa 600

gaaaaaacaa atttaaatac gggcctgaca gctgcacaag ttaagcaaaa aattgaagct 660

ggtgaaatta ataaggccgt tgatgatcaa tttaaaacaa ataaacaaat tattgcagaa 720

aatttattta cttactttaa cttgattttc ttagtactat cattactttt gatttttgtt 780

ggcgcatata aggatttaac ttttttgcca gtcattgttt taaatactgt aattggaatt 840

gtgcaagaaa ttcgtgctaa aaaaatattg aacaagttaa acgtaatgaa tgctacagat 900

attggggctt tgcgagatgg aaaagaagta caagtaccaa tagaagagtt agttaaaggc 960

gatattgttt tattaaaaac aggcgatcag attccagcag 1000

<210> 7
<211> 1000
<212> DNA
<213> Lactobacillus plantarum WCFS1

<400> 7
gaggccgcaa tgattgcatt gatatgggca gaagatcaaa atggcctgat tggcaaccag 60

ggccagcttc cctggcattt accagcagac atgcagcggt tcaaagcgct gacgaccgga 120

caccatgtcg tgatggggcg caagactttt gcgggcttta aacgcccgct cccacggcgg 180

accaattggg tgctctcacg tcagtctgat ttaaagttgc caccggaagt ccatcaacta 240

gcagatgtgg cggcgatcca aacactcgcg gccgcccatc cggatgaacc gatttttgtc 300

attggtggtg cggtggtgtt tgaagccgtg ttaccggtgg ccgattattt atatcggacg 360

cgtatcaacg caaggtttga tggtgatact tggatgccgg ccgtggatta cacgcagtgg 420

cagctggtga gccaacaaat tgggacggtg gatgagaaaa atcaatatcc gtacgaattt 480

gatgatttcc gccgtcgtta attcagtggc aaccgtgaaa ctcaggcaga tttctgctat 540

actgaaagca ttaattttat tgtaacgatc gtttaaacgt tacaagtata agaaagcgtg 600

gcaaagtatg acgaaaatca agattgtgac cgattcttcg gcaaatttga cggatgccga 660

ggtaaaaaaa tacgatatta ccgtgattcc gttgactgtc atgatcgatg gcaccattta 720

cgtggaagat gaaacgatta cgcgtgaaga atttattgac aagatggcaa cggctaaatc 780

tttaccaaag acgagccaac cggcactcgg aacgttcatt gaaacgtttg ataagttagg 840

tgcggatggc gcgagtgtga tttgtatcaa tatgctcgaa gccatttcag gaacagtgca 900

cacggcggaa caagcggctt cgattacgaa gaccgatgtc accgtgatcg acgctcggac 960

gaccgaccgt gcgatggcct ttcaagtttt gactgctgct 1000

<210> 8

<211>  1000
<212>  DNA
<213>  Lactobacillus salivarius UCC118

<400>  8
tctgtgaggt gtgcactatg atttcatttg tatgggcaga agatcaaaaa catcaaatag      60

gatataaagg acatttacct tggagacttc cagccgattt ggctcatttt aaagaagtta     120

caatgggtca tccaatggta atggggaaga aaacgtttga tagttttcct ggtttattac     180

caagacgtca acatatagtg ttaacccatg atactaactt agaagaaaag tataaagata     240

atccccaagt agaaattatg aattctattg atgaattaac ttcttggtta gatgaaaatc     300

agtttcaaga agttagcgtt attggtggag cgatgttatt caacttactt cttaataaag     360

tagataaatt atataagact gaaatactaa gtgagtttaa tggcgatacg gttatgccta     420

ctattaatta tgatgaattt aaacttgttt caaaaaaaat cggtaaggta gatgaaaaaa     480

acaagtatcc ttatgtattt ttggaatacg aaagaaaata atttctaata aaaataatga     540

aagatgagta agacaatttt gtagtactca tcttttttta catttttaag gcttaaatgt     600

tgacttcaag ttaacttgaa gttttattat agatatgtaa gttcatatag ggttgatatt     660

aaaaactcta aatttttaag agaaggatgt gctaaatatg caaaaattat caagtttaac     720

atcgccatta gttctagcga atggtgttga aattccagga ctaggctatg gtacttacca     780

aactccacct gaagatacat acaaagtagt atcagaagca ttatcaatcg gttatcgcca     840

cattgacaca gctgcattat atggcaatga aagtggtgta ggtaaggctg ttaaagatag     900

tgggttgaag cgtgaagaag tatttattac tagtaagtta tggaatactg aacgtggata     960

cgacaaagca attgcttcct ttaataagac tttagaaaat                          1000


<210>  9
<211>  27
<212>  PRT
<213>  Lactococcus lactis subspecies Cremoris MG 1363

<400>  9

Met Lys Lys Lys Ile Ile Ser Ala Ile Leu Met Ser Thr Val Ile Leu
1               5                   10                  15


Ser Ala Ala Ala Pro Leu Ser Gly Val Tyr Ala
            20                  25

## Description

The present invention relates to recombinant microbes that, in comparison to the wild type ancestor, show reduced capacity of colonization to the mucosa, in particular when residing in the alimentary tract of a human or animal. In particular, the invention relates to the engineering and use of colonizing microorganisms with reduced colonizing capacity, e.g. as host organisms for expression and delivery of therapeutic proteins and peptides.

### Field of the Invention

The invention is in the field of use of colonizing microbes (such as, for example, *Escherichia coli, Lactobacillus* sp, *Streptococcus* sp., *Bacteroides* sp., *Bifidobacterium* sp., yeast, fungi...) in human and veterinary medicine. Medical applications can be a consequence of inherent features of such microbe, or such microbe can be genetically modified (GM) for the administration of prophylactic or therapeutic peptides or proteins to humans or animals suffering from or at a risk of developing a disease, or such GM microbes may be used for vaccination purposes.

More in particular, the invention relates to GM microbes that show reduced capacity of colonization, in particular when residing in the human or animal mucosa (such as, without limitation, alimentary tract, oral cavity, nasal cavity, urogenital tract, etc.) as part of a treatment or vaccination of a human or animal.

In a preferred embodiment, the GM microbe contains an inactive thymidylate synthase gene that causes the reduced capability for said microbe to colonize in human or animal mucosa.

In a more preferred embodiment, the GM microbe contains an inactive thymidylate synthase gene that completely prevents the GM microbe to colonize in human or animal mucosa .

### Background of the Invention

Both wild type as well as GM microbes are gaining importance in medical applications in humans and animals where applications using prophylactic as well as therapeutic microbes are described in pre-clinical and clinical settings.

Selected microbes (also called functional microflora, probiotics) can be used for the prevention or alleviation of pathologies.

Lactic acid bacteria but also other species are increasingly becoming important as hosts for recombinant expression of heterologous polypeptides *in vitro* (e.g., US 5,559,007) as well as for *in vivo* or *in situ* expression and delivery of antigens and/or therapeutically relevant polypeptides (e.g., WO 97/14806).

Lactic acid bacteria, and particularly *Lactobacillus* and *Lactococcus,* are considered as GRAS-microorganisms (i.e., generally regarded as safe) and may thus be relatively readily administered to humans and animals.

Because *Lactococcus lactis* was the first lactic acid bacteria species characterised into great detail at a molecular and genetic level, it enjoyed an increasing interest as production host for heterologous proteins and eventually as *in situ* delivery system for biologically active molecules. *L. lactis* is non-pathogenic, non-invasive and non-colonizing.

Compared to expression systems in non-colonizing microorganisms however, the use of colonizing microorganisms may facilitate protein expression and delivery *in situ,* via prolonged retention and closer contact to the host tissues. The potency of any therapeutic application may therefore benefit substantially from using a colonizing microorganism.

As an example, the more robust character of the *Lactobacillus* bacteria may prefer its use as a medicament over the *Lactococcus* bacteria.

However, the disadvantage of using colonizing instead of non-colonizing microbes (such as using *Lactobacillus* sp instead of *Lactococcus*) as a host strain for delivering therapeutic proteins or peptides to human or animal is the unwanted colonization of the mucosa, in particular of the alimentary tract, by the prophylactic or therapeutic strain, which makes it rather difficult to completely stop the treatment after cure (or in case of side effects) or to establish a dose response relationship. Indeed, timing and dosage will intrinsically be more difficult to perform when using a colonizing microorganism as when compared to using a non-colonizing microorganism. Sometimes, the only possibility to stop the treatment may be the use of selective antibiotics, which should however be avoided due to development of antibiotic resistance, impact on other microflora and other reported side effects. Moreover, it may be more difficult to environmentally contain a colonizing microorganism as it will be unclear for how long the microorganisms will remain attached to the mucosal surface, which complicates the regulatory and biosafety considerations with such organisms.

Said disadvantages clearly complicate the use of non-recombinant selected microbes and has prevented until now that recombinant colonizing strains have been used and approved for clinical trials. For these reasons, colonizing microorganisms with reduced colonization capacity could hold even more advantages, including more precise control on timing and dosage of protein expression, as well as a clear view on residence time and environmental containment, which are considered critical factors for deliberate release of any genetically modified organisms (GMO).

A few authors have investigated the effect of a thymidylate synthase gene (ThyA) mutation on the colonization capacity of bacteria (Bigas et al., 2006 (Int Microbiol 9(4): 297-301) (Besier et al., 2007 (Int J Med Microbiol 297(4): 217-25). The results of these studies are contradictive and only relate to pathogenic bacteria and the relation of ThyA to the remaining pathogenicity. In clinical settings one has observed even that thymidine-auxotrophic small colony variants of *Staphylococcus aureus* are able to persist in patients suffering from chronic airway infections and have been implicated in persistent, relapsing and treatment-resistant infections (Proctor et al., 1995 (Clin Infect Dis 20(1): 95-102) (Spanu et al., 2005 (Clin Infect Dis 41(5): e48-52). Besier et al (2007) has even shown that defects in the ThyA function are causative for the formation of the thymidine-auxotrophic small colony variants of *Staphylococcus aureus.* Another author, Hasselbring et al. (Hasselbring et al., 2006 (J Bacteriol 188(17): 6335-45), has screened *Mycoplasma pneumonia* mutants to identify genes associated with gliding but dispensable for cytadherence, which resulted in a number of genes (including ThyA) suggesting to be involved in the gliding motility of *Mycoplasma pneumonia.*

Other authors have used ThyA gene mutations for biological containment of genetically modified *Lactococcus sp.* and *Lactobacillus sp.,* in order to avoid the surviving and spreading of the genetically modified organisms in the environment (Steidler et al., 2003 (Nat. Biotechnol. 21(785-9), WO2004/046346, WO2005/111194). The experiments in these disclosures are mainly related to growth and survival of said recombinant bacteria and did not yield any information on the colonization capacity of the *Lactobacillus* ThyA mutants nor did they give any direction to a possible use of ThyA gene impairment in *Lactobacillus* to reduce or avoid colonization. Instead, given that some of these publications indicated the ability of *Lactobacillus* ThyA mutants to produce and deliver therapeutic proteins *in situ,* one would have no reason to expect said bacteria to display reduced colonization.

### Summary of the Invention

It is the objective of the present invention to obtain a suitable system to reduce or avoid the colonization capacity of prophylactic and therapeutic microbes at the mucosa, in particular in the alimentary tract.

We have surprisingly found that a defect in the function of ThyA in otherwise colonizing microbes (e.g. *Lactobacillus sp., Streptococcus* sp. or *Bacteroides* sp.) causes a reduced colonization capacity and in a preferred setting a complete prevention of colonization of these strains in the mucosa (in particular in the alimentary tract). Hence, rendering defective a thyA gene of a microorganism, such as an endogenous thyA gene and more particularly an endogenous chromosomally-located thyA gene of a microorganism, allows to reduce or abolish the latter's colonizing capacity.

A first embodiment of the invention is an isolated strain of a microbe comprising a defective thymidylate synthase (thyA) gene. Preferably, the thyA gene may be rendered defective by means of recombinant DNA technology, such that the defective thyA gene may be denoted as recombinant defective thyA gene, and by extension a recombinant microbe. Preferably disclosed is the isolated strain of the microbe comprising the defective thyA gene, and in particular the defective recombinant thyA gene, wherein said isolated strain of the microbe has a reduced capacity of colonizing a mucosa (e.g., one or more mucosa loci) of a human or animal in comparison to the wild-type microbe, such as a wild-type ancestor of the isolated strain.

Preferably, said defective thyA gene, and particularly said defective recombinant thyA gene, is situated in the chromosome and is inactivated by gene disruption. Preferably, said defective thyA gene, and particularly said defective recombinant thymidylate synthase gene, is a non-reverting mutant gene.

Further preferably, the isolated strain of the microbe comprising the defective thyA gene as taught herein may be a thymidylate synthase deficient strain, i.e., the strain may be deficient in thymidylate synthase activity, such as for example may display a substantial reduction in or more preferably absence of thymidylate synthase activity. Hence, phenotypically said isolated strain may preferably manifest as a thymine and/or thymidine auxotrophic strain, i.e., requiring exogenously provided thymine and/or thymidine for survival, growth and/or propagation. Hence, preferably in the isolated strain of the microbe comprising the defective thyA gene as taught herein, said defect in the thyA gene and consequently in thymidylate synthase activity is not complemented by another, active (non-defective) thyA gene.

Further preferably, the isolated strain may be derived from a microbe which is normally (i.e., when thymidylate synthase proficient, in other words when comprising an active thyA gene) colonizing, i.e., capable of colonizing a mucosa, such as human or animal mucosa, preferably mucosa or the alimentary tract.

In another aspect of the present invention, the microbe is a yeast or fungi, particularly a recombinant yeast or fungi, in particular any yeast or fungi capable of surviving in the mammalian intestine. Alternatively, said yeast or fungi may have a known probiotic capacity, such as without limitation yeast or fungi strains selected from kefir, kombucha or dairy products.

In a particular embodiment, said yeast or fungi, particularly said recombinant yeast or fungi, is selected from the group consisting of *Candida sp., Aspergillus sp., Penicillium sp., Saccharomyces sp., Hansenula sp., Kluyveromyces sp. Schizzosaccharomyces sp. Zygosaccharomyces sp., Pichia sp., Monascus sp., Geotrichum sp* and *Yarrowia sp.* More in particular, said yeast is *Saccharomyces cerevisiae,* even more in particular said yeast is *Saccharomyces cerevisiae* subspecies *boulardii.*

In a further embodiment, the isolated strain comprising defective thyA gene as taught herein may be of a microbe which is a bacterium, more preferably a non-pathogenic and/or non-invasive bacterium, yet more preferably a Gram-positive bacterium, in particular such bacterium capable of residing in mucosa of human or animal, in particular in the mucosa of the alimentary tract, such as a normally (i.e., when thymidylate synthase proficient) colonizing bacterium. Exemplary bacterial species include without limitation, *Bacteroides sp, Clostridium sp., Fusobacterium sp., Eubacterium sp., Ruminococcus sp., Peptococcus sp., Peptostreptococcus sp., Streptococcus* sp., *Bifidobacterium sp., Escherichia sp.,* and *Lactobacillus sp.*

Another embodiment of the invention is an isolated strain of a gram-positive bacterium that is capable of residing in the mucosa, in particular in the mucosa of the alimentary tract, such as *Bacteroides sp, Clostridium sp., Eubacterium sp., Ruminococcus sp., Peptococcus sp., Peptostreptococcus sp., Streptococcus sp., Bifidobacterium sp.* and *Lactobacillus sp.,* wherein such gram-positive bacterium comprises a defective thyA gene, in particular a defective recombinant thymidylate synthase gene (thyA). Preferably, said defective thyA gene, and particularly said defective recombinant thyA gene, is situated in the chromosome and inactivated by gene disruption. Preferably, said defective thyA gene, and particularly said defective recombinant thymidylate synthase gene, is a non-reverting mutant gene.

In a preferred embodiment the invention is an isolated strain of *Lactobacillus sp.* comprising a defective thyA gene, in particular a defective recombinant thymidylate synthase gene (thyA). Preferably, said defective thyA gene, and particularly said defective recombinant thyA gene, is situated in the chromosome and is inactivated by gene disruption. Preferably, said defective thyA gene, and particularly said defective recombinant thymidylate synthase gene, is a non-reverting mutant gene. Preferably, said *Lactobacillus sp.* is a *Lactobacillus plantarum* strain, *Lactobacillus acidophilus* or *Lactobacillus rhamnosus* strain; even more preferably, said *Lactobacillus* is a *Lactobacillus salivarius* strain or a *Lactobacillus casei* strain; and any subspecies and strains thereof.

In a preferred embodiment the invention is an isolated strain of *Bacteroides sp.* comprising a defective thyA gene, in particular a defective recombinant thymidylate synthase gene (thyA). Preferably, said defective thyA gene, and particularly said defective recombinant thyA gene, is situated in the chromosome and is inactivated by gene disruption. Preferably, said defective thyA gene, and particularly said defective recombinant thymidylate synthase gene, is a non-reverting mutant gene. *Bacteroides sp.* are highly suitable for delivery of prophylactic and/or therapeutic molecules such as proteins to subjects (see, e.g., (Hamady et al., 2009 (Gut) Epub ahead of print). Preferably, said *Bacteroides sp.* is a *Bacteroides ovatus* strain, and any subspecies and strains thereof.

In a further preferred embodiment the invention is an isolated strain of *Streptococcus sp.* comprising a defective thyA gene, in particular a defective recombinant thymidylate synthase gene (thyA). Preferably, said defective thyA gene, and particularly said defective recombinant thyA gene, is situated in the chromosome and is inactivated by gene disruption. Preferably, said defective thyA gene, and particularly said defective recombinant thymidylate synthase gene, is a non-reverting mutant gene. Preferably, said *Streptococcus sp.* is *Streptococcus mutans.* According, the microbe, strain or host cell as intended herein may preferably be a *Streptococcus sp* more preferably *Streptococcus mutans* and any subspecies and strains thereof, comprising a defective thyA gene.
*Streptococcus mutans* is a lactic acid bacterium that normally colonizes dental surfaces, and as such an may be particularly suitable to serve as a host organism for delivery of molecules as taught herein to the oral mucosa.
*S. mutans* constitutes a phenotypically homogeneous group of colonizing, Gram-positive *Streptococci* (Hamada et al., 1980 (Microb rev 44(2): 331-84). *S. mutans* is acidogenic and acidoduric, non-motile and facultative anaerobic. At present, *S. mutans* is commonly divided into four serotypes, based on the chemical composition of its cell surface rhamnose-glucose polymers (Hamada et al., 1980 (Microb rev 44(2): 331-84; Maruyama et al., 2009 (BMC genomics 10(358). In this view, serotype c is dominant among *S. mutans* clinical isolates (almost 80%) and is now considered the ancestral phenotype, with other serotypes having evolved strain-specific genes (Maruyama et al., 2009 (BMC genomics 10(358).The natural habitat of *S. mutans* is the human mouth, with a clear preference for tooth surfaces as well as prosthetic devices (Hamada et al., 1980 (Microb rev 44(2): 331-84). The organism can also be isolated from feces, in humans (Kilian et al., 1971 (Archives of oral biology 16(5): 553-4; Finegold et al., 1975 (Can res 35(11 Pt. 2): 3407-17; Liljemark et al., 1978 (J dent resh 57(2): 373-9; Hamada et al., 1980 (J clin microbiol 11(4): 314-8; Unsworth, 1980 (J hyg 85(1): 153-64) as well as rats (Huber et al., 1977 (J of dent res 56(12): 1614-9; Thomson et al., 1979 (Caries res 13(1): 9-17). Although the bacterium appears not to be widely distributed in wild animals, *S. mutans* has among others been isolated from oral surfaces of several monkey and bat species (Lehner et al., 1975 (Nature 254(5500): 517-20; Coykendall et al., 1976 (Infection and immunity 14(3): 667-70; Dent et al., 1978 (Journal appl bact 44(2): 249-58; Beighton et al., 1982 (Archives of oral biology 27(4): 331-5), rats (Lehner et al., 1975 (Nature 254(5500): 517-20; Coykendall et al., 1976 (Infection and immunity 14(3): 667-70; Hamada et al., 1980 (Microbio rev 44(2): 331-84), hamsters (Gehring et al., 1976 (Deutsche zahnarztliche Zeitschrift 31(1): 18-21; Hamada et al., 1980 (Microb rev 44(2): 331-84), macropods (Samuel, 1982 (Archives of oral biology 27(2): 141-6) and *Beagle* dogs (Wunder et al., 1976 (J dent res 55(6): 1097-102).
The oral microflora is a complex ecosystem of microbial species, and without proper oral hygiene, large microbial masses and biofilms (plaques) may develop on dental surfaces. Although the causative relationship between specific oral bacterial species and dental caries has been the subject of many studies, *Streptococci* normally comprise the majority of the total viable cell count retrieved from human carious lesions (Hamada et al., 1980 (Microb rev 44(2): 331-84).
The adherence of *S. mutans* and other oral bacteria to tooth surfaces and the formation of dental plaque are of major significance in the development of dental caries. These processes are complex and involve a variety of bacterial and host components. Bacterial attachment to the tooth surface is usually preceded by the formation of a thin layer of heterogeneous salivary glycoproteins (pellicle), which facilitates the adhesion of *S*. *mutans.*
*S. mutans* is capable of metabolizing (fermenting) a wide variety of carbohydrates. The acidification of the local environment by the end product of metabolism (i.e., lactic acid) inhibits many competing bacterial species, thus enabling *S. mutans* to maintain its niche, while at the same time causing dental demineralization in the host. Importantly, while the fermentation of any available carbohydrate could lead to lactic acid and damage to the dental enamel, sucrose is particularly important in this process because it also serves as substrate for extracellular enzymes which synthesize sucrose-derived polymers. These extracellular polymers (glucans) consist solely of glucose units and possess a marked ability to promote adherence when synthesized *de novo* on various solid surfaces (Hamada et al., 1980 (Microb rev 44(2): 331-84).
Several approaches to reduce *S. mutans* colonization have been described, focusing mainly on dental plaque-related diseases. These interventions range from the obvious mechanical cleansing, to compounds targeting bacterial interactions, the salivary pellicle or bacterial polymers adsorbed to the tooth (i.e. glucans), as well as strategies aimed at therapeutic manipulation of the oral microflora (Liljemark et al., 1978 (J dent res 57(2): 373-9; Allaker et al., 2009 (Int J Antimicrob Agents 33(1): 8-13), such as for example using probiotics such as specific *Lactobacillus* strains, or using replacement therapy with less cariogenic or even impaired *S. mutans* strains, such as for example via deletion (and if necessary, compensation) of the genetic sequence encoding lactate dehydrogenase (e.g., WO/1996/040865) (Allaker et al., 2009 (Int J Antimicrob Agents 33(1): 8-13). Such clones produce no detectable lactic acid and are significantly less cariogenic. In this view however, the genetic modification is to maintain or even improve upon the colonizing potential of the engineered *S. mutans* strains, to replace the wild type *S*. *mutants* bacteria, which is distinct from the goal of the present invention, i.e., to obtain strains such as *S. mutans* strains having reduced or abolished colonization capacity.

A "non-reverting mutant" as used throughout this specification means that the reversion frequency is lower than 10⁻⁸, preferably the reversion frequency is lower than 10⁻¹⁰, even more preferably, said reversion frequency is lower than 10⁻¹², even more preferably, said reversion frequency is lower than 10⁻¹⁴, most preferably, said reversion frequency is not detectable using the routine methods known to the person skilled in the art.

The main advantage of the invention is the improved use of the selected or recombinant microbes (e.g. *Lactobacillus sp., Bacteroides sp.,* or *Streptococcus* sp.) as a medicament to treat animals or humans suffering from a disease, or its use as a vaccine, to the extent that it is now possible to have a controlled dosing of the prophylactic and/or therapeutic microbes. This can avoid the use of antibiotics in case of terminating the treatment.
Prophylactic and/or therapeutic traits may be inherent to the strain comprising the defective ThyA (e.g., probiotic microbes) and/or may be expressed as a consequence of genetic engineering of the strain comprising a defective ThyA .
Accordingly, the invention also provides an isolated strain of a microbe comprising the defective thyA gene as taught herein, wherein said strain elicits a prophylactic and/or therapeutic effect in a subject, preferably in a human or animal. Also provided is an isolated strain of a microbe comprising the defective thyA gene as taught herein, wherein the strain further expresses an expression product, preferably a heterologous expression product, particularly a prophylactically and/or therapeutically relevant expression product (such as an expression product capable of eliciting a prophylactic and/or therapeutic response in a subject, preferably in a human or animal subject), such as for example a (preferably, heterologous) peptide, polypeptide or protein, and more particularly antigens and/or non-vaccinogenic prophylactically and/or therapeutically active peptides, polypeptides or proteins. To achieve the expression of said heterologous expression product(s), the strain may commonly comprise a recombinant nucleic acid encoding the heterologous expression product(s). Advantageously, the recombinant nucleic acid may comprise suitable regulatory sequence(s) (e.g., a promoter) operably linked to one or more open reading frames encoding the heterologous expression product(s).

Therefore, another aspect of the invention is the use of an isolated strain of the microbe comprising the defective thyA gene as taught herein, and particularly the use of *Lactobacillus* strain, *Streptococcus* strain (e.g., *Streptococcus mutans* strain) or *Bacteroides* strain (e.g., *Bacteroides ovatus* strain), comprising the defective thyA gene according to the invention, as a reduced colonizing, or even preferably non-colonizing, strain for the delivery of prophylactic and/or therapeutic molecules, such as for the delivery of one or more (preferably heterologous) expression products as taught herein. Preferably, delivery using a *Streptococcus* strain (e.g., *Streptococcus mutans*) may be to oral mucosa. The delivery of prophylactic and/or therapeutic molecules has been disclosed, as a non-limiting example, in WO 97/14806 and in WO 98/31786. Prophylactic and/or therapeutic molecules include, but are not reduced to polypeptides such as insulin, growth hormone, prolactine, calcitonin, group 1 cytokines, group 2 cytokines, group 3 cytokines, neuropeptides and antibodies (or functional fragments thereof), and polysaccharides such as polysaccharide antigens from pathogenic bacteria. In a preferred embodiment, the thyA gene of an isolated strain of the microbe as taught herein, such as particularly of a *Lactobacillus sp.* strain, *Streptococcus* sp. strain or *Bacteroides* sp. strain, preferably *Lactobacillus salivarius* or *Lactobacillus casei,* or *Streptococcus mutans,* or *Bacteroides ovatus,* is disrupted and replaced by a functional human interleukin-10 expression cassette and the strain can be used for delivery of IL-10. Said interleukin-10 expression unit is preferably, but not reduced to, a human interleukin-10 expression unit or gene encoding for human interleukin-10. Therefore, a preferred embodiment is the use of an isolated strain of the microbe comprising the defective thyA gene as taught herein, and particularly the use of a *Lactobacillus sp.* or *Streptococcus* sp. (e.g., *Streptococcus mutans* strain) or *Bacteroides* sp. (e.g., *Bacteroides ovatus* strain),comprising the defective thyA gene strain according to the invention to deliver human interleukin-10. Methods to deliver said molecules and methods to treat diseases such as inflammatory bowel diseases are explained in detail in WO 97/14806 and WO 00/23471 and Steidler et al. 2000 (Steidler et al., 2000 (Science 289; 1352-5) that are hereby incorporated by reference. The present invention demonstrates that the strain according to the invention surprisingly passes the gut at more or less the same speed as the non-colonizing *Lactococcus* strains and show that their loss of colonization capacity results in a much faster clearance of the gut after the last administration than wild type *Lactobacillus sp.*
Another aspect is the use of an isolated strain of the microbe comprising the defective thyA gene as taught herein, and particularly the use of *Lactobacillus* strain or *Streptococcus* strain (e.g., *Streptococcus mutans* strain) or *Bacteroides* strain (e.g., *Bacteroides ovatus* strain), comprising the defective thyA gene according to the invention, as a reduced colonizing probiotic strain, or even preferably non-colonizing probiotic strain.

Another aspect of the invention is a pharmaceutical composition, comprising an isolated strain of the microbe comprising the defective thyA gene as taught herein, and particularly comprising a *Lactobacillus* sp. or *Streptococcus* sp. (e.g., *Streptococcus mutans* strain) or *Bacteroides* sp. (e.g., *Bacteroides ovatus* strain), comprising the defective thyA gene such as a *Lactobacillus* sp. or *Streptococcus* sp. or *Bacteroides* sp. thyA disruption mutant, according to the invention, preferably as a reduced colonizing, or even more preferably non-colonizing strain. The strain may optionally and preferably further express one or more (preferably heterologous) expression products as taught herein. As a non-limiting example, the bacteria may be encapsulated to improve the delivery to the intestine. Methods for encapsulation are known to the person, skilled in the art, and are disclosed, amongst others, in EP0450176. Said pharmaceutical composition may typically comprise one or more suitable excipients, and may further optionally comprise one or more additional active ingredients beneficial for the particular disease or condition to be treated. Further useful compositions comprising the isolated strain of the microbe comprising the defective thyA gene as taught herein may include inter alia starter cultures, innocula, lyophilized compositions, frozen liquid compositions, and food and feed compositions, any of which may comprise additional components common in the art for such compositions.

Still another aspect of the invention is the use of a strain according to the invention, preferably as a reduced colonizing, or even preferably non-colonizing strain, for the preparation of a medicament. Further disclosed is the strain according to the invention, preferably as a reduced colonizing, or even preferably non-colonizing strain, for use as a medicament. Also contemplated is the use of a strain according to the invention, preferably as a reduced colonizing, or even preferably non-colonizing strain, for the preparation of a vaccine. The strain may optionally and preferably further express one or more (preferably heterologous) expression products as taught herein. Hence, also contemplated is the strain as taught herein expressing (and capable of delivering) one or more (preferably heterologous) expression products for use in treating a disease or condition in which the administration of said one or more (preferably heterologous) expression products is capable of eliciting a prophylactic and/or therapeutic effect. Also contemplated is the strain as taught herein expressing (and hence, capable of delivering) one or more (preferably heterologous) expression products for use in delivery of said expression product(s) to a human or animal; and use of said strain for the manufacture of a medicament for delivering said expression product(s) to a human or animal. Preferably, said medicament is used to treat Crohn's disease or inflammatory bowel disease, oral mucositis, lesions of gastro-intestinal tract, autoimmune pathologies, allergy, metabolic disorders such as obesity and diabetes, etc.

Further disclosed is a method for reducing or abolishing the colonization capacity of a microbe, comprising rendering defective a thymidylate synthase (thyA) gene in said microbe. Hereby, the method produces a strain of the microbe having a reduced capacity of colonizing the mucosa of a human or animal in comparison to the wild-type microbe, such as a wild-type ancestor of said strain. Preferred microbe types and species may be as enumerated above. Said rendering defective of the thyA gene may preferably be by means of recombinant DNA technology. Preferably, the thyA gene may be situated in the chromosome and may be rendered defective by gene disruption. Preferably, the thyA gene may be rendered defective non-revertingly.
Preferably, the rendering defective of the thyA gene results in thymidylate synthase deficiency, i.e., a substantial reduction in or more preferably absence of thymidylate synthase activity in the so-modified microbe, such as in particular results in thymine and/or thymidine auxotrophy in said microbe.
Further preferably, the microbe subjected to the method may be normally (i.e., when thymidylate synthase proficient, in other words when comprising an active thyA gene, prior to rendering the same defective) colonizing, i.e., capable of colonizing a mucosa, such as human or animal mucosa, preferably mucosa or the alimentary tract. In particular embodiments, the microbe may be as taught here above.

Preferably, the microbe may elicit a prophylactic and/or therapeutic effect in a subject, preferably in a human or animal. Preferably, the microbe may express an expression product, preferably a heterologous expression product, particularly a prophylactically and/or therapeutically relevant expression product (such as an expression product capable of eliciting a prophylactic and/or therapeutic response in a subject, preferably in a human or animal subject), such as for example a (preferably heterologous) peptide, polypeptide or protein, and more particularly antigens and/or non-vaccinogenic prophylactically and/or therapeutically active peptides, polypeptides or proteins. To provide for the expression of said heterologous expression product(s), the microbe may commonly comprise a recombinant nucleic acid encoding the (preferably heterologous) expression product(s). Advantageously, the recombinant nucleic acid may comprise suitable regulatory sequence(s) (e.g., a promoter) operably linked to one or more open reading frames encoding the (preferably heterologous) expression product(s). Hence, also disclosed is a method for reducing or abolishing the colonization capacity of a microbe, wherein said microbe expresses a (preferably heterologous) expression product, said method comprising rendering defective a thymidylate synthase (thyA) gene in said microbe.

A related aspect thus provides the use of thymidylate synthase deficiency in a microbe for reducing or abolishing the colonization capacity of said microbe.

These and further aspects and preferred embodiments of the invention are described in the following sections and in the appended claims.

### BRIEF DESCRIPTION OF FIGURES

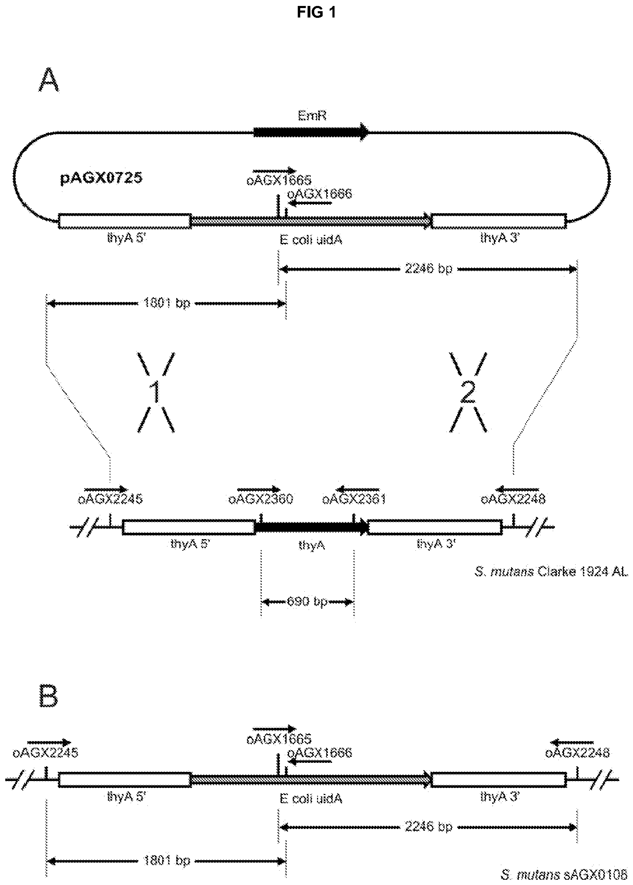

**Figure 1** illustrates generation of thyA- uidA+ *S. mutans* strain sAGX0108. All binding sites of primers oAGX1665, oAGX1666, oAGX2245, oAGX2248, oAGX2360 and oAGX2361are indicated. Note that primers oAGX2245 and oAGX2248 only bind to chromosomal sequences outside of the target areas for recombination and do not bind to pAGX0725. Sizes of PCR products are indicated as bp.
   Panel A: The non-replicative plasmid pAGX0725 was introduced in *S. mutans* strain Clarke 1924 AL through electroporation. Plating on erythromycin and X-gluc containing solid agar plates allowed for the selection of blue erythromycin resistance marker (EmR)+ colonies that have undergone homologous recombination with the bacterial chromosome either upstream (thyA 5'; 1) or downstream (thyA 3'; 2) of thyA. Clones are selected that have undergone recombination at both regions. This leads to the removal of thyA from and the insertion of uidA into the bacterial chromosome. Both homologous recombinations were screened for by PCR using either the primer pair oAGX2245/oAGX1666 (thyA 5') or oAGX1665/oAGX2248 (thyA 3'). Presence or absence of thyA was revealed by PCR using primer pair oAGX2360/oAGX2361.
   Panel B: Final structure of the modified thyA locus of *S. mutans* strain sAGX0108. The presence of uidA in the *S. mutans* sAGX0108 is demonstrated by PCRs using primer pairs oAGX2245/oAGX1666 (showing co-linearity between uidA and the chromosome upstream) and oAGX1665/oAGX2248 (showing co-linearity between uidA and the chromosome downstream). Presence or absence of thyA is revealed by PCR using primer pair oAGX2360/oAGX2361.
   Genomic structures and binding of primers are based on published *(S. mutans* Clarke 1924 AL, assumed to be identical to NCBI Reference Sequence NC_004350.1) or predicted (*S*. *mutans* sAGX0108: in the NCBI Reference Sequence NC_004350.1 the thyA gene was replaced with the *E. coli* uidA gene, GenBank: CP001396.1; complement position 1584343..1586154) DNA sequences.

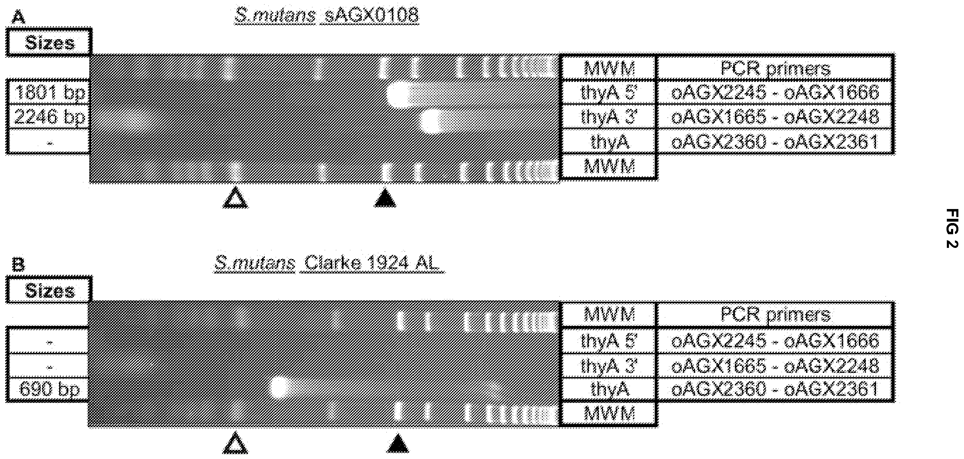

**Figure 2** illustrates PCR analysis of the thyA locus of thyA- *S. mutans* sAGX0108 and its parent thyA+ *S. mutans* Clarke 1924 AL.
   Panel A: PCR reactions performed on DNA extracts from *S. mutans* sAGX0108 using the indicated primers (PCR primers) show the presence of appropriately sized products overlapping both thyA 5' (thyA 5') and thyA 3' (thyA 3') cross over regions while no PCR product corresponding the thyA gene (thyA) can be detected. Primers oAGX1665 and oAGX1666 bind the uidA gene so both PCR reactions that use these primers provide evidence for the presence of the uidA gene.
   Panel B: PCR reactions performed on DNA extracts from *S. mutans* Clarke 1924 AL using the indicated primers show the absence of products overlapping both thyA 5' and thyA 3' cross over regions while an appropriately sized PCR product corresponding to the thyA gene can be detected.
   Sizes indicate the expected size of individual PCR products using the indicated primers and published *(S. mutans* Clarke 1924 AL: assumed to be identical to NCBI Reference Sequence NC_004350.1) or predicted (*S*. *mutans* sAGX0108: in NCBI Reference Sequence NC_004350.1 the thyA gene was replaced with the *E. coli* uidA gene, GenBank: CP001396.1; complement bp position 1584343..1586154) DNA sequences.
   MWM: Molecular weight markers, contains the following discrete bands (in base pairs): 75, 134, 154, 201, 220, 298, 344, 396, 506, 517 (Δ), 1018, 1636 (▲), 2036, 3054, 4072, 5090, 6108, 7126, 8144, 9162, 10180, 11198, 12216.

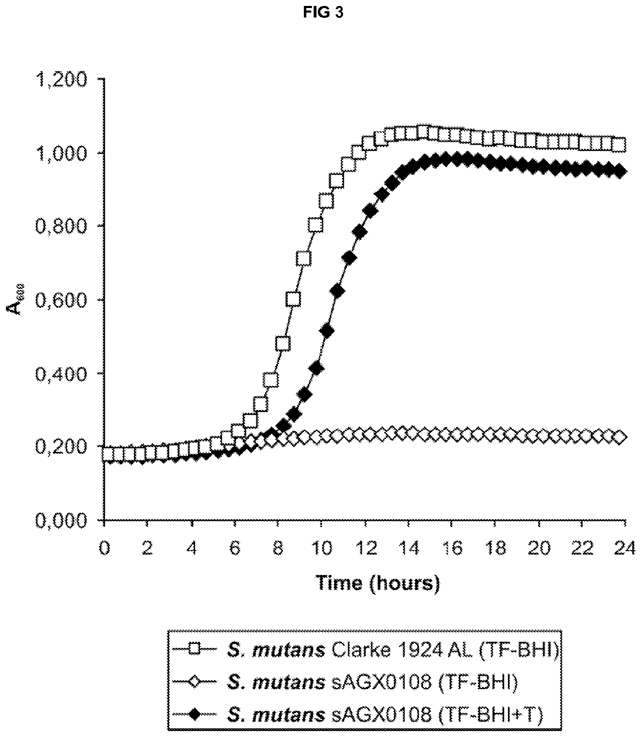

**Figure 3** illustrates growth of WT *S. mutans* Clarke 1924 AL and thyA- *S. mutans* sAGX0108 as monitored by automated measurement of absorbance at 600 nm (A₆₀₀). Cultures were grown in thymidine free Brain Heart Infusion broth (TF-BHI). While *S. mutans* Clarke 1924 AL shows growth to complete saturation, no growth could be observed for *S. mutans* sAGX0108 in the absence of thymidine. The addition of thymidine (+T) to TF-BHI cultures of *S. mutans* sAGX0108 complements its growth deficiency and supports growth to saturation. All data presented are averages of measurements performed on three individual cultures grown in a Bioscreen C MBR automated turbidimeter. Absorbance values at 0 hours reflect A₆₀₀ of TF-BHI.

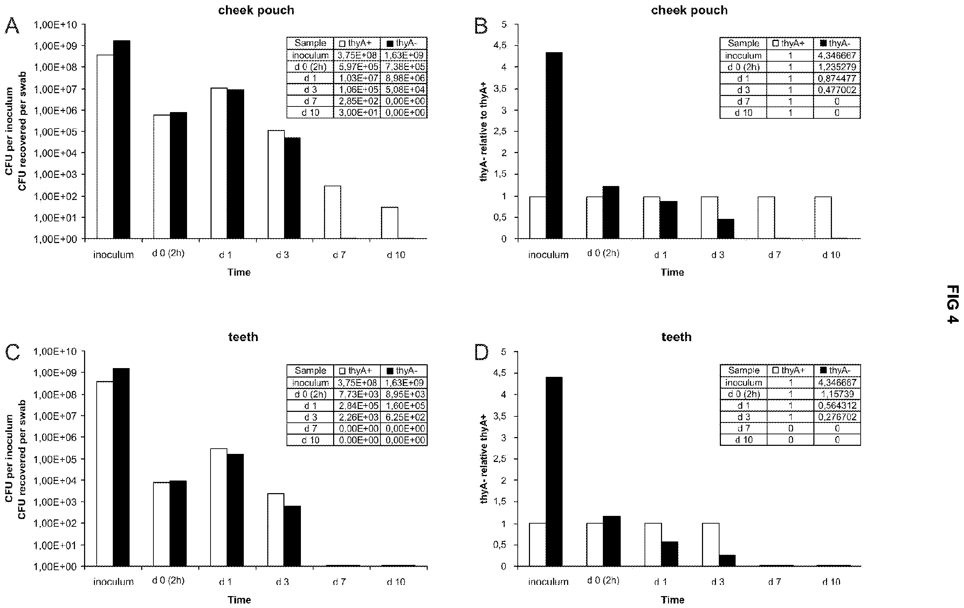

**Figure 4** illustrates relative colonization of thyA+ *(S. mutans* Clarke 1924 AL pILPOL; white bars) and thyA- *(S. mutans* sAGX0108; black bars) *S. mutans* in the oral cavity of hamsters. The entire dental surface of hamsters was cleaned with cotton swabs immediately prior to inoculation with *S*. *mutans.* Saturated overnight cultures of *S. mutans* Clarke 1924 AL pILPOL and *S. mutans* sAGX0108 were concentrated 50x in BAM9T. Of both strains, concentrations were determined and presented as #CFU per inoculum (inoculum). The concentrated suspensions were mixed 50:50 and hamsters were inoculated in the left cheek pouch with 50 µl of the bacterial suspension. Using cotton swabs, samples were taken from the left cheek pouch (cheek) and entire dental surface (teeth) 2 hours (d 0 (2h)), 1 day (d 1), 3 days (d 3), 7 days (d 7) and 10 days (d 10) subsequent to inoculation. 8 hamsters were used in this experiment. Hamsters were used for sampling cheek pouch and dental surface at one time point only. At 2 hours, 1 day and 3 days, samples were taken from 2 hamsters and averages were calculated. At day 7 and 10 samples were taken from 1 hamster. Cotton tips holding the samples were cut off the shaft, submerged in 1 ml of 1xM9 and mixed thoroughly. To determine the concentrations of *S. mutans* Clarke 1924 AL pILPOL and *S. mutans* sAGX0108 present on the swabs, sample suspensions were diluted appropriately in 1xM9 and plated in duplicate on BHI solid agar plates containing erythromycin and BHI solid agar plates containing X-gluc and thymidine respectively.
   Panel A shows #CFU of both strains in the inoculum (CFU per inoculum) and in samples from the left cheek pouch, taken at the different time points (CFU recovered per swab). Panel B shows the relative presence of thyA- vs thyA+ *S. mutans* (set as 1) in the inoculum and in samples from cheek pouches taken at the different time points. Panel C shows #CFU of both strains in the inoculum (CFU per inoculum) and in samples from the entire dental surface, taken at the different time points (CFU recovered per swab). Panel D shows the relative presence of thyA- vs thyA+ *S. mutans* (set as 1, except when no thyA+ were recovered) in the inoculum and in samples from dental surfaces taken at the different time points. For all panels, exact values are shown in the inserts.

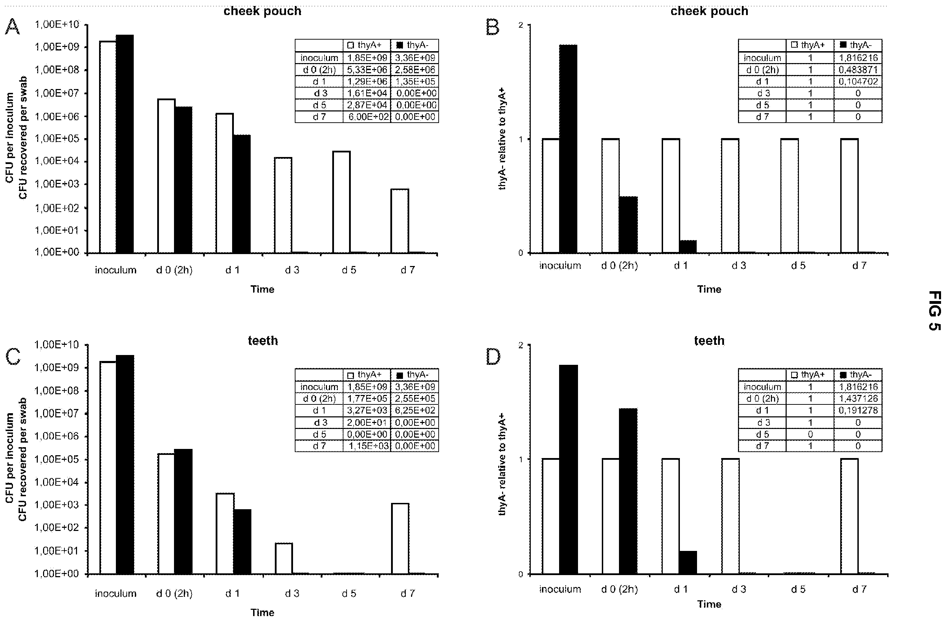

**Figure 5** illustrates relative colonization of thyA+ *(S. mutans* Clarke 1924 AL pILPOL Cm+; white bars) and thyA- *(S. mutans* sAGX0108 Cm+; black bars) *S. mutans* in the oral cavity of hamsters. The entire dental surface of hamstes was cleaned with cotton swabs immediately prior to inoculation with *S*. *mutans.* Saturated overnight cultures of *S. mutans* Clarke 1924 AL pILPOL Cm+ and *S. mutans* sAGX0108 Cm+ were concentrated 50x in BAM9T. Of both strains, concentrations were determined and presented as #CFU per inoculum (inoculum). The concentrated suspensions were mixed 50:50 and hamsters were inoculated in the left cheek pouch with 50 µl of the bacterial suspension. Using cotton swabs, samples were taken from the left cheek pouch (cheek) and entire dental surface (teeth) 2 hours (d 0 (2h)), 1 day (d 1), 3 days (d 3), 5 days (d 5) and 7 days (d 7) subsequent to inoculation. 8 hamsters were used in this experiment. Hamsters were used for sampling cheek pouch and dental surface at one time point only. At 2 hours, 1 day and 3 days, samples were taken from 2 hamsters and averages were calculated. At day 5 and 7 samples were taken from 1 hamster. Cotton tips holding the samples were cut off the shaft, submerged in 1 ml of 1xM9 and mixed thoroughly. To determine the concentrations of *S. mutans* Clarke 1924 AL pILPOL Cm+ and *S. mutans* sAGX0108 Cm+ present on the swabs, sample suspensions were diluted appropriately in 1xM9 and plated on BHI solid agar plates containing erythromycin and BHI solid agar plates containing X-gluc and thymidine respectively.
   Panel A shows #CFU of both strains in the inoculum (CFU per inoculum) and in samples from the left cheek pouch, taken at the different time points (CFU recovered per swab). Panel B shows the relative presence of thyA- vs thyA+ *S. mutans* (set as 1) in the inoculum and in samples from cheek pouches taken at the different time points. Panel C shows #CFU of both strains in the inoculum (CFU per inoculum) and in samples from the entire dental surface, taken at the different time points (CFU recovered per swab). Panel D shows the relative presence of thyA- vs thyA+ *S. mutans* (set as 1, except when no thyA+ were recovered) in the inoculum and in samples from dental surfaces taken at the different time points. For all panels, exact values are shown in the inserts.

### DETAILED DESCRIPTION OF THE INVENTION

As used herein, the singular forms "a", "an", and "the" include both singular and plural referents unless the context clearly dictates otherwise. By way of example, "a cell" refers to one or more than one cell.

The terms "comprising", "comprises" and "comprised of" as used herein are synonymous with "including", "includes" or "containing", "contains", and are inclusive or open-ended and do not exclude additional, non-recited members, elements or method steps. The term also encompasses "consisting of".

The recitation of numerical ranges by endpoints includes all numbers and fractions subsumed within that range, as well as the recited endpoints.

The term "about" as used herein when referring to a measurable value such as a parameter, an amount, a temporal duration, and the like, is meant to encompass variations of and from the specified value, in particular variations of +/-20% or less, preferably +/-10% or less, more preferably +/-5% or less, even more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

All documents cited in the present specification are hereby incorporated by reference in their entirety. In particular, the teachings of all documents herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, ensuing definitions are included to better appreciate the teaching of the present invention.

The term "nucleic acid" as used herein means a polymer of any length composed essentially of nucleotides, e.g., deoxyribonucleotides and/or ribonucleotides. Nucleic acids can comprise purine and/or pyrimidine bases and/or other natural (e.g., xanthine, inosine, hypoxanthine), chemically or biochemically modified (e.g., methylated), non-natural, or derivatised nucleotide bases. The backbone of nucleic acids can comprise sugars and phosphate groups, as can typically be found in RNA or DNA, and/or one or more modified or substituted sugars and/or one or more modified or substituted phosphate groups. The term "nucleic acid" further preferably encompasses DNA, RNA and DNA/RNA hybrid molecules, specifically including hnRNA, pre-mRNA, mRNA, cDNA, genomic DNA, amplification products, oligonucleotides, and synthetic (e.g. chemically synthesised) DNA, RNA or DNA/RNA hybrids. A "nucleic acid" can be double-stranded, partly double stranded, or single-stranded. Where single-stranded, the nucleic acid can be the sense strand or the antisense strand. In addition, nucleic acid can be circular or linear.

In a preferred embodiment, a nucleic acid may be DNA or RNA, more preferably DNA.

The term "recombinant nucleic acid" refers generally to a nucleic acid which is comprised of segments joined together using recombinant DNA technology. When a recombinant nucleic replicates in a host organism, the progeny nucleic acids are also encompassed within the term "recombinant nucleic acid".

Standard reference works setting forth the general principles of recombinant DNA technology include Molecular Cloning: A Laboratory Manual, 2nd ed., vol. 1-3, ed. Sambrook et al., Cold Spring Harbor Laboratory Press, Cold Spring Harbor, N.Y., 1989; Current Protocols in Molecular Biology, ed. Ausubel et al., Greene Publishing and Wiley-Interscience, New York, 1992 (with periodic updates); Innis et al., PCR Protocols: A Guide to Methods and Applications, Academic Press: San Diego, 1990. General principles of microbiology are set forth, for example, in Davis, B. D. et al., Microbiology, 3rd edition, Harper & Row, publishers, Philadelphia, Pa. (1980).

Expression of peptides, polypeptides and proteins can be achieved through operably linking nucleic acid sequences or open reading frame(s) (ORFs) encoding said products with regulatory sequences allowing for expression of the nucleic acids or ORFs, e.g., in the microbes and/or strains taught herein. Such expression may be achieved, e.g., under suitable (culture) conditions or upon addition of inducers (e.g., where inducible regulatory sequences are used).

An "operable linkage" is a linkage in which regulatory sequences and sequences sought to be expressed are connected in such a way as to permit said expression. For example, sequences, such as, e.g., a promoter and an ORF, may be said to be operably linked if the nature of the linkage between said sequences does not: (1) result in the introduction of a frame-shift mutation, (2) interfere with the ability of the promoter to direct the transcription of the ORF, (3) interfere with the ability of the ORF to be transcribed from the promoter sequence.

The precise nature of regulatory sequences or elements required for expression may vary between expression environments, but typically include a promoter and a transcription terminator, and optionally an enhancer.

Reference to a "promoter" or "enhancer" is to be taken in its broadest context and includes transcriptional regulatory sequences required for accurate transcription initiation and where applicable accurate spatial and/or temporal control of gene expression or its response to, e.g., internal or external (e.g., exogenous) stimuli. By "promoter" is meant generally a region on a nucleic acid molecule, preferably DNA molecule, to which an RNA polymerase binds and initiates transcription. A promoter is preferably, but not necessarily, positioned upstream, i.e., 5', of the sequence the transcription of which it controls. A promoter may optionally comprise an operator configured to control transcription from the promoter. As used herein, the term "operator" refers to a nucleotide sequence, preferably DNA sequence, which controls the initiation and/or maintenance of transcription of a sequence from a promoter.

The term "microbes" generally refers to microorganisms and particularly refers to bacteria, yeast, fungus. The term "colonizing microbes" refers to those bacteria, yeast and fungi that are capable of residing in the mucosa of the animal or human subject, in particular in the mucosa of the alimentary tract. Hence, the term "colonizing microbes" would be readily understood by a skilled person, in particular to encompass microbes that are able to survive, grow and remain in a given site such as a mucosa for extended periods of time. The colonizing nature or colonization capacity of a microbe may be determined by means available in the art, such as by analyzing biopsy samples or mucus samples in order to determine persistence of microbes in said samples, or taking stool samples to evaluate excretion of microbes.

While having a clear denotation per se, the term "reducing" with reference to the colonization capacity may in particular refer to any qualitative and/or quantitative alteration, change or variation which decreases or diminishes the colonizing character of a microbe. By means of example and not limitation, manifestations of reduced colonizing capacity may include a comparably lower titer of microbe persisting at a given site and/ or a comparably faster clearance of a microbe from a given site, etc.

The term encompasses any extent of such reduction. Where said reduction can be monitored in terms of a quantifiable variable (e.g., the titer of a microbe in a sample from a given site, e.g., at one or more time points) the "reduction" may particularly encompass a decrease in the value of said variable by at least about 10%, e.g., by at least about 20%, preferably by at least about 30%, e.g., by at least about 40%, more preferably by at least about 50%, e.g., by at least about 60%, even more preferably by at least about 70%, e.g., by at least about 80%, still more preferably by at least about 90%, e.g., by at least about 95%, such as by at least about 96%, 97%, 98%, 99% or even by 100%, compared to a reference microbe.

Further, conform its common meaning, the term "abolish" as used herein particularly encompasses removing or eliminating the colonizing capacity of a microbe such that the so-modified microbe would be deemed to have become non-colonizing.

Examples of such colonizing microbes include but are not limited to *Candida sp., Aspergillus sp., Penicillium sp., Saccharomyces sp., Hansenula sp., Kluyveromyces sp. Schizzosaccharomyces sp. Zygosaccharomyces sp., Pichia sp., Monascus sp., Geotrichum sp, Yarrowia sp. Bacteroides sp, Clostridium sp., Fusobacterium sp., Eubacterium sp., Ruminococcus sp., Peptococcus sp., Peptostreptococcus sp., Streptococcus sp., Bifidobacterium sp., Escherichia sp.* and *Lactobacillus sp.*

The term *"Bifidobacterium"* or *"Bifidobacterium sp."* generally refers to the genus *Bifidobacterium* and encompasses any taxon (e.g., species, subspecies, strain) classified as belonging to such in the art. By means of example, *Bifidobacterium* or *Bifidobacterium sp.* includes the species *B*. *adolescentis, B. angulatum, B. animalis, B*. *asteroides, B. bifidum, B. boum, B. breve, B. catenulatum, B. choerinum, B. coryneforme, B. cuniculi, B. denticolens, B. dentium, B. gallicum, B. gallinarum, B. indicum, B. infantis, B. inopinatum, B. lactis, B. longum, B. magnum, B. merycicum, B. minimum, B. pseudocatenulatum, B. pseudolongum, B. pullorum, B. ruminantium, B. saeculare, B. subtile, B. suis, B. thermacidophilum* and *B*. *thermophilum.*

The term *"Lactobacillus"* or *"Lactobacillus sp."* generally refers to the genus *Lactobacillus* and encompasses any taxon (e.g., species, subspecies, strain) classified as belonging to such in the art. By means of example, *Lactobacillus* or *Lactobacillus sp.* includes the species *L. acetotolerans, L. acidifarinae, L. acidipiscis, L. acidophilus, L. agilis, L. algidus, L. alimentarius, L. amylolyticus, L. amylophilus, L. amylotrophicus, L. amylovorus, L. animalis, L. antri, L. apodemi, L. aviarius, L. bifermentans, L. brevis, L. buchneri, L. camelliae, L. casei, L. catenaformis, L. ceti, L. coleohominis, L. collinoides, L. composti, L. concavus, L. coryniformis, L. crispatus, L. crustorum, L. curvatus, L. delbrueckii, L. delbrueckii subsp. bulgaricus, L. delbrueckii subsp. lactis, L. diolivorans, L. equi, L. equigenerosi, L. farraginis, L. farciminis, L. fermentum, L. fornicalis, L. fructivorans, L. frumenti, L. fuchuensis, L. gallinarum, L. gasseri, L. gastricus, L. ghanensis, L. graminis, L. hammesii, L. hamsteri, L. harbinensis, L. hayakitensis, L. helveticus, L. hilgardii, L. homohiochii, L. iners, L. ingluviei, L. intestinalis, L. jensenii, L. johnsonii, L. kalixensis, L. kefiranofaciens, L. kefiri, L. kimchii, L. kitasatonis, L. kunkeei, L. leichmannii, L. lindneri, L. malefermentans, L. mali, L. manihotivorans, L. mindensis ,,L. mucosae, L. murinus, L. nagelii, L. namurensis, L. nantensis, L. oligofermentans, L. oris, L. panis, L. pantheris, L. parabrevis, L. parabuchneri, L. paracollinoides, L. parafarraginis, L. parakefiri, L. paralimentarius, L. paraplantarum, L. pentosus, L. perolens, L. plantarum, L. pontis, L. psittaci, L. rennini, L. reuteri, L. rhamnosus, L. rimae, L. rogosae, L. rossiae, L. ruminis, L. saerimneri, L. sakei, L. salivarius, L. sanfranciscensis, L. satsumensis, L. secaliphilus, L. sharpeae, L. siliginis, L. spicheri, L. suebicus, L. hailandensis, L. ultunensis, L. vaccinostercus, L. vaginalis, L. versmoldensis, L. vini, L. vitulinus, L. zeae, L. zymae* and any subspecies and strains thereof. In preferred embodiments of the invention the *Lactobacillus* is *Lactobacillus casei or. Lactobacillus plantarum* or *Lactobacillus rhamnosus.* In further preferred embodiments of the invention the *Lactobacillus* is *Lactobacillus salivarius.*

The term *"Bacteroides"* or *"Bacteroides sp."* generally refers to the genus *Bacteroides* and encompasses any taxon (e.g., species, subspecies, strain) classified as belonging to such in the art. By means of example, *Bacteroides* or *Bacteroides sp.* includes the species *B*. *acidifaciens, B. distasonis, B. gracilis, B. eggerthii, B. fragilis, B. oris, B*. *ovatus, B. putredinis, B. pyogenes, B. stercoris, B. suis, B*. *tectus, B. thetaiotaomicron, B. uniformis, B. vulgatus,* and any subspecies and strains thereof. In preferred embodiments of the invention the *Bacteroides* is *Bacteroides ovatus.*

The term *"Streptococcus"* or *"Streptococcus* sp." generally refers to the genus *Streptococcus* and encompasses any taxon (e.g., species, subspecies, strain) classified as belonging to such in the art. By means of example, *Streptococcus* or *Streptococcus* sp. includes the species *S*. *agalactiae, S*. *anginosus, S. bovis, S. canis, S. equi, S. iniae, S. mitis, S*. *mutans, S. oralis, S. parasanguinis, S. peroris, S. pneumoniae, S*. *pyogenes, S*. *ratti, S. salivarius, S. salivarius* ssp. *thermophilus, S. sanguinis, S. sobrinus, S*. *suis, S. uberis, S. vestibularis, S. viridans,* and any subspecies and strains thereof. Preferably the *Streptococcus* is *Streptococcus mutans.*

The term "thymidylate synthase" is common in the art and generally refers to the enzyme EC 2.1.1.45, also known under the synonymous denotations dTMP synthase, thymidylate synthetase, methylenetetrahydrofolate:dUMP C-methyltransferase and TMP synthetase. Thymidylate synthase activity in particular involves catalyzing reaction of 5,10-methylenetetrahydrofolate + dUMP to dihydrofolate + dTMP. A thymidylate synthase may be generally encoded by and expressed from a thymidylate synthase gene, abbreviated as "thyA" gene. By means of example, the term "thymidylate synthase (*thyA*) gene of *Lactobacillus"* denotes a gene encoding the said enzyme in a *Lactobacillus.* The sequence of the *thyA* gene from several *Lactobacillus* taxons has been described, such as, e.g., from *Lactobacillus delbrueckii* subsp. *Bulgaricus* (Sasaki et al., 2004 (Appl Environ Microbiol 70(3): 1858-64), *Lactobacillus casei* (Genbank Gene ID: 4419806). A skilled person is capable of identifying and isolating *thyA* gene homologues from further taxons of *Lactobacillus.*

In the present disclosure, the thymidylate synthase (thyA) gene may be rendered defective or inactive in the microbe. Rendering a thyA gene defective or inactive may in particular involve modifying or altering the thyA gene such that the thymidylate synthase activity expressed in the microbe from said thyA gene is reduced or preferably abolished.

Whereas a thyA gene defect as intended herein may lead to any extent of reduction of thymidylate synthase activity expressed in the microbe from said thyA gene, particularly useful may be a substantial reduction of the thymidylate synthase activity in the microbe, such as for example a reduction by at least about 30%, e.g., by at least about 40%, preferably by at least about 50%, e.g., by at least about 60%, more preferably by at least about 70%, e.g., by at least about 80%, even more preferably by at least about 90%, e.g., by at least about 95%, such as by at least about 96%, 97%, 98%, 99% or even by 100%, compared to a non-defective (active) thyA gene. Preferably, the thyA gene defect may result in no thymidylate synthase activity being produced from said thyA gene, i.e., in abolishment of said activity. The activity of thymidylate synthase may be measured, without limitation, by established enzymatic assays or by assessing the degree of thymine and/or thymidine auxotrophy of a microbe.

A thyA gene in a microbe may be rendered defective, for example, by suitably altering the regulatory sequences controlling the thyA expression and/or by suitably altering the sequences (ORF) coding for the thymidylate synthase.

Preferably, the alteration may involve gene disruption. The term "gene disruption" may commonly encompass genetic alterations that reduce or preferably abolish the encoded gene product activity, and in particular gene disruption, as used throughout this specification, includes disruption by insertion of a DNA fragment, disruption by deletion of the gene, or a part thereof, as well as exchange of the gene or a part thereof by another DNA fragment, and said disruption is induced by recombinant DNA techniques, and not by spontaneous mutation. Preferably, disruption is the exchange of the gene, or a part thereof, by another functional gene.

In an embodiment, the microbe as disclosed herein may possess inherent prophylactic and/or therapeutic traits, such as for example, the microbe may be deemed a probiotic. The term "probiotic" is know in the art and may particularly encompass microbial food or feed supplements whose primary aim is to maintain and/or improve the health and/or well-being of a subject such as a human or animal. Beneficial effects of probiotics may in particular be due to improving mucosal microbial balance, such as microbial balance in the alimentary tract or parts thereof.

In a further embodiment, the microbe may express a heterologous expression product. The term "heterologous" when referring to the relationship between an expression product and a microbe means that said expression product is not normally expressed by said microbe in nature. In particular, expression of said expression product by the microbe may be created using recombinant DNA techniques, more in particular through introducing to the microbe a recombinant nucleic acid encoding and effecting the expression of said expression product in said microbe.

As used herein, the term "antigen" generally refers to a substance foreign to a body (esp. to a body of a human or animal subject whereto the antigen is to be administered) that evokes an immune response, including humoral immunity and/or cellular immunity response, and that is capable of binding with a product, e.g., an antibody or a T cell, of the immune response. Hence, in a preferred example, an antigen requires a functioning immune system of a subject to which it is administered to elicit a physiological response from such a subject.

An antigen according to the invention may be derived from any polypeptide to which an immune response in a human or animal subject would be therapeutically useful, e.g., from a pathogen, e.g., from a viral, prokaryotic (e.g., bacterial) or eukaryotic pathogen, from a non-physiological protein (e.g., a protein derived from cancer tissue), from allergen (e.g., for eliciting immune tolerance), etc.

Hence, in a preferred embodiment an antigen is capable of eliciting an immune tolerance response in a subject such as a human or animal.

The term "alimentary canal" is known in the art and may particularly encompass the mouth, oesophagus, stomach, small intestine (including inter alia Duodenum, Jejunum and Ileum) and large intestine (colon) rectum and anus. The phrase "mucosa of the alimentary canal" may refer to mucosa of any one or more or all sites of the alimentary canal, as may be apparent from the context.

The term "a non-vaccinogenic therapeutically active polypeptide" refers generally to a polypeptide that, in a human or animal subject to which it is administered, does not elicit an immune response against itself and is able to achieve a therapeutic effect. Hence, the therapeutic effect of such a polypeptide would be expected to be directly linked to its own natural biological function whereby it can achieve particular effects in a body of a subject; rather than producing a therapeutic effect by acting as an immunogenic and/or immunoprotective antigen in the subject. Hence, the non-vaccinogenic therapeutically active polypeptide should be biologically active in its expressed form or, at least, must be converted into the biologically active form once released from the expressing host cell. Preferably, such biologically active form of the said polypeptide may display a secondary and preferably also tertiary conformation which is the same or closely analogous to its native configuration.

Preferably, the non-vaccinogenic therapeutically active polypeptide is also non-toxic and non-pathogenic.

In a preferred embodiment, the non-vaccinogenic therapeutically active polypeptide may be derived from human or animal, and may preferably correspond to the same taxon as the human or animal subject to which it is to be administered.

Non-limiting examples of suitable non-vaccinogenic therapeutically active polypeptides include ones which are capable of functioning locally or systemically, e.g., is a polypeptide capable of exerting endocrine activities affecting local or whole-body metabolism and/or the biologically active polypeptide(s) is/are one(s) which is/are capable of the regulation of the activities of cells belonging to the immunohaemopoeitic system and/or the one or more biologically active polypeptides is/are one(s) which is/are capable of affecting the viability, growth and differentiation of a variety of normal or neoplastic cells in the body or affecting the immune regulation or induction of acute phase inflammatory responses to injury and infection and/or the one or more biologically active polypeptides is/are one(s) which is/are capable of enhancing or inducing resistance to infection of cells and tissues mediated by chemokines acting on their target cell receptors, or the proliferation of epithelial cells or the promotion of wound healing and/or the one or more biologically active polypeptides modulates the expression or production of substances by cells in the body.

Specific examples of such polypeptides include, without limitation, insulin, growth hormone, prolactin, calcitonin, luteinising hormone, parathyroid hormone, somatostatin, thyroid stimulating hormone, vasoactive intestinal polypeptide, cytokines (including but not limited to interleukins IL-2, IL-3, IL-4, IL-5, IL-6, IL-7, IL-9, IL-10,IL-11, IL-12, IL-13, IL-17 any of IL-14 to IL-35), GM-CSF, M-CSF, SCF, IFNs, EPO,G-CSF, LIF, OSM, CNTF, GH, PRL, the TNF family of cytokines, e.g., TNFα, TNPβ, CD40, CD27 or FAS ligands, the IL-1 family of cytokines, the fibroblast growth factor family, the platelet derived growth factors, transforming growth factors and nerve growth factors, the epidermal growth factor family of cytokines, the insulin related cytokines, etc. Alternatively, the therapeutically active polypeptide can be a receptor or antagonist for the therapeutically active polypeptides as defined above. Further specific examples of such suitable polypeptides are listed, e.g., in WO 96/11277, page 14, lines 1-30, incorporated herein by reference; in WO 97/14806, page 12, line 1 through page 13, line 27, incorporated herein by reference; or US 5,559,007, col. 8, line 31 through col. 9, line 9, incorporated by reference herein. In an embodiment, said non-vaccinogenic therapeutically active polypeptide may be hIL-10, GLP-2, GLP-1, TFF or hPYY.

Accordingly, in an embodiment the microbe as taught herein may comprise a recombinant nucleic acid which encodes an antigen and/or a non-vaccinogenic prophylactically and/or therapeutically active polypeptide, wherein the said antigen is capable of eliciting an immune response, preferably protective immune response or immune tolerance response, in a human or animal subject, and/or the said non-vaccinogenic therapeutically active polypeptide is capable of producing a prophylactic and/or therapeutic effect in a human or animal subject.

WO 97/14806 further specifically discloses co-expression of antigens with immune response stimulatory molecules, such as, e.g., interleukins, e.g., IL-2 or IL-6, by bacteria. Accordingly, such co-expression of two or more antigens and/or non-vaccinogenic prophylactically and/or therapeutically active polypeptides in a microbe as taught herein is also contemplated.

In a further preferred embodiment, the open reading frame encoding a (preferably heterologous) expression product further comprises a sequence encoding a secretion signal in phase with a polypeptide encoded by the ORF. This advantageously allows for secretion of the expressed polypeptide from the host cell and thereby may facilitate, e.g., isolation or delivery.

Typically, a secretion signal sequence represents an about 16 to about 35 amino acid segment, usually containing hydrophobic amino acids that become embedded in the lipid bilayer membrane, and thereby allow for the secretion of an accompanying protein or peptide sequence from the host cell, and which usually is cleaved from that protein or peptide. Preferably, the secretion signal sequence may be so-active in a host cell intended for use with the nucleic acid comprising the said signal sequence, e.g., a bacterial host cell, preferably a lactic acid bacterium, more preferably *Lactobacillus.*

Secretion signal sequences active in suitable host cells are known in the art; exemplary *Lactobacillus* signal sequences include those of usp45 (see, US 5,559,007), usp45N4 (see WO 2008/084115) and others, see, e.g., (Perez-Martinez et al., 1992 (Mol Gen Genet 234(3): 401-11); (Sibakov et al., 1991 (Appl Environ Microbiol 57(2): 341-8). Preferably, the signal sequence is located between the promoter sequence and the ORF, i.e. the signal sequence is located 3' from the promoter sequence and precedes the ORF of the polypeptide of interest. In a preferred embodiment, the signal sequence encodes the amino acid sequence MKKKIISAIL MSTVILSAAA PLSGVYA (usp45) (SEQ ID NO: 9).

The recombinant nucleic acid may comprise a promoter, being a native promoter from a microbe (e.g., *Lactobacillus, Streptococcus, Bacteroides,* or *Lactococcus* species) or a functional variant or functional fragment thereof, operably linked to one or more open reading frames encoding a (preferably heterologous) expression product.

The promoter may be chosen from the group comprising or consisting of the native promoters of genes of *Lactococcus* for (see, e.g., WO 2008/084115): 1) DNA-directed RNA polymerase, beta' subunit / 160 kD subunit (*rpoC*), 2) DNA-directed RNA polymerase, beta subunit / 140 kD subunit (*rpoB*), 3) non-heme iron-binding ferritin (*dpsA*), 4) pyruvate kinase (*pyk*), 5) glutamyl-tRNA synthetases (*gltX*)*,* 6) phosphopyruvate hydratase (*eno*), 7) glutamine synthetase (*glnA*) 8) glutamine synthetase repressor (*glnR*), 9) dipeptidase PepV (*pepV*), 10) F0F1-type ATP synthase beta subunit (ATP synthase F1 beta subunit) (*atpD*), 11) 3-phosphoglycerate kinase (*pgk*), 12) glyceraldehyde-3-phosphate dehydrogenase (*gapB*), 13) acetate kinase (*ackA*), 14) 3-oxoacyl-(acyl-carrier-protein) synthase II (*fabF*), 15) 3-ketoacyl-(acyl-carrier-protein) reductase (*fabG or fabG1*), 16) DNA-directed RNA polymerase, alpha subunit / 40 kD subunit (*rpoA*), 17) Proline dipeptidase (*pepQ*), 18) fructose-bisphosphate aldolase (*fbaA*), 19) ribosomal protein S4 (*rpsD*), 20) superoxide dismutase (sodA), 21) ribosomal protein S12 (*rpsL*) and ribosomal protein S7 (*rpsG*)*,* 22) ribosomal protein L18 (*rpIR*) and ribosomal protein S5 (*rpsE*) and ribosomal protein L30/L7E (*rpmD*), 23) S-ribosylhomocysteinase (*luxS*)*,* 24) ribosomal protein L19 (*rpIS*), 25) ribosomal protein S11 (*rpsK or infA*)*,* 26) ribosomal protein L10 (*rplJ*)*,* 27) ribosomal protein L7/L12 (*rpIL*), 28) HU-like DNA-binding protein (*hllA*), 29) 50S ribosomal protein L28 (*rpmB*), 30) phosphotransferase system IIB component (*ptcB*), 31) F0F1-type ATP synthase alpha subunit (*atpA*), 32) multiple sugar-binding transport ATP-binding protein (*msmK*), 33) pyruvate dehydrogenase E1 component alpha subunit (*pdhA*), 34) cell division protein (*difIVA or ftsA*), 35) UDP-galactopyranose mutase (*glf1*)*,* 36) glutamyl aminopeptidase (*pepA*), 37) predicted dehydrogenase related protein (*llmg_0272*)*,* 38) ribosomal protein S2 (*rpsB*), 39) translation initiation factor 3 (IF-3) (*infC*), 40) ribosomal protein L4 (*rplD*) and ribosomal protein L23 (*rplW*) and ribosomal protein L2 (*rplB*)*,* 41) Phenylalanyl-tRNA synthetase beta chain (*pheT*), 42) transcription elongation factor GreA (*greA*), 43) ATP-dependent Clp protease proteolytic subunit (*clpP*)*,* 44) ribosomal protein L15 (*rplO*), 45) ribosomal protein L11 (*rplK*)*,* 46) ribosomal protein S8 (*rpsH*), 47) ribosomal protein L21 (*rplU),* 48) ribosomal protein S13 (*rpsM*), 49) ribosomal protein S19 (*rpsS*) and ribosomal protein L22 (*rplV*) and ribosomal protein L16 (*rpIP*) and ribosomal protein L14 (*rplN*)*,* 50) ribosomal protein S10 (*rpsJ*), 51) co-chaperonin GroES (Hsp10) (*groES*), 52) ribosomal protein L24 (*rplX*) and 53) putative holiday junction resolvase (*llmg_0151*) and functional variants and functional fragments of the said native promoters.

The promoter may be 28) bacterial nucleoid DNA-binding protein / HU-like DNA-binding protein (*hlla* or *hup*), even more preferably, said promoter may be the PhllA promoter.

The promoter may be 3) non-heme iron-binding ferritin (*dpsA* or *LACR_2311*)*,* promoter 9) dipeptidase PepV (*pepV* or *LACR_0908*)*,* or promoter 20) superoxide dismutase (sodA or *LACR_0458*)*,* respectively, even more preferably, said promoter may be the PdpsA, PpepV or PsodA promoter.

In embodiments, the recombinant nucleic acid may comprise:
(a) PdpsA, usp45 and hIL-10; PdpsA, usp45N4 and hIL-10;
   PpepV, usp45 and hIL-10; PpepV, usp45N4 and hIL-10;
   PsodA, usp45 and hIL-10; PsodA, usp45N4 and hIL-10;
   PhllA, usp45 and hIL-10; PhllA, usp45N4 and hIL-10;
(b) PdpsA, usp45N4 and hTFF1; PdpsA, usp45 and hTFF1;
   PpepV, usp45N4 and hTFF1; PpepV, usp45 and hTFF1;
   PsodA, usp45N4 and hTFF1; PsodA, usp45 and hTFF1;
   PhllA, usp45N4 and hTFF1; PhllA, usp45 and hTFF1;
(c) PdpsA, usp45N4 and hTFF3; PdpsA, usp45 and hTFF3;
   PpepV, usp45N4 and hTFF3; PpepV, usp45 and hTFF3;
   PsodA, usp45N4 and hTFF3; PsodA, usp45 and hTFF3;
   PhllA, usp45N4 and hTFF3; PhllA, usp45 and hTFF3;
(d) PdpsA, usp45N4 and hPYY; PdpsA, usp45 and hPYY;
   PpepV, usp45N4 and hPYY; PpepV, usp45 and hPYY;
   PsodA, usp45N4 and hPYY; PsodA, usp45 and hPYY;
   PhllA, usp45N4 and hPYY; PhllA, usp45 and hPYY; PhllA, usp45 and hPYY G9 (3-36);
(e) PdpsA, usp45N4 and GLP-1; PdpsA, usp45 and GLP-1;
   PpepV, usp45N4 and GLP-1; PpepV, usp45 and GLP-1;
   PsodA, usp45N4 and GLP-1; PsodA, usp45 and GLP-1;
   PhllA, usp45N4 and GLP-1; PhllA, usp45 and GLP-1;
(f) PdpsA, usp45N4 and GLP-2; PdpsA, usp45 and GLP-2;
   PpepV, usp45N4 and GLP-2; PpepV, usp45 and GLP-2;
   PsodA, usp45N4 and GLP-2; PsodA, usp45 and GLP-2;
   PhllA, usp45N4 and GLP-2; or PhllA, usp45 and GLP-2.

The promoter may in the alternative be chosen from the group comprising or consisting of the native promoters of genes of *Lactobacillus,* preferably but without limitation of *Lactobacillus rhamnosus,* for 1) ribosomal protein S14 and ribosomal protein S17 and ribosomal protein L15 and ribosomal protein S3 (*rpsJ*), 2) nucleoid DNA-binding protein (*dnabp*), 3) ribosomal protein S21 (*rpS21*), 4) 50S ribosomal protein L19 (*rplS*), 5) 50S ribosomal protein L17 (*rnap40*)*,* 6) 50S ribosomal protein L13 (*rplM*), 7) phosphoglycerate mutase 1 (*pgm1*)*,* 8) ribosomal protein S4 (*rpS4*), 9) glyceraldehyde-3-phosphate dehydrogenase (*cggr*), and functional variants and functional fragments of the said native promoters. Said promoters can ensure particularly strong expression of molecules of interest. Said promoters may find particular use in expression of molecules of interest in *Lactobacillus* sp. such as for example in *L. plantarum, L. acidophilus, L. rhamnosus, L. salivarius* or *L. casei,* and any subspecies and strains thereof.

The promoter may in the alternative be chosen from the group comprising or consisting of the native promoters of genes of *Streptococcus,* preferably but without limitation of *Streptococcus mutans,* for 1) 30S ribosomal protein S10, 2) 50S ribosomal protein L27, 3) 30S ribosomal protein S15, 4) 30S ribosomal protein S16, 5) 50S ribosomal protein L19, 6) 30S ribosomal protein S8, 7) 50S ribosomal protein L18 and 30S ribosomal protein S5, 8) 30S ribosomal protein S9, 9) 50S ribosomal protein L17, 10) 30S ribosomal protein S13, 11) 30S ribosomal protein S7, 12) 50S ribosomal protein L15, 13) 30S ribosomal protein S4, 14) 50S ribosomal protein L6, 15) 30S ribosomal protein S3 and 50S ribosomal protein L3, 16) phosphoglyceromutase, and functional variants and functional fragments of the said native promoters. Said promoters can ensure particularly strong expression of molecules of interest. Said promoters may find particular use in expression of molecules of interest in *Streptococcus* sp. such as for example in *S. mutans,* and any subspecies and strains thereof.

By means of example, the above promoters may be chosen from the group comprising or consisting of nucleic acids set forth in Tables 1 and Table 2 below, and functional variants and functional fragments of the said native promoters. In said tables, The Gene ID numbers uniquely identify the said genes in the "Entrez Gene" database of NCBI (www.ncbi.nlm.nih.gov/entrez/query.fcgi?db=gene) as described in Maglott et al. 2005. (Entrez Gene: gene-centered information at NCBI. Nucleic Acids Res. 33: D54-D58). The NCBI Reference Sequence accession numbers in said Tables provide particular nucleic acid sequence information. Tables 1 and 2 further identify regions of the nucleic acids which may be considered as respective promoter regions.

**Table 1. Select genes and promoter regions identified in Lactobacillus rhamnosus (particularly L. rhamnosus HN001).**

| **GI-number** | **Gene / Protein name** | **Abbrev.** | **Locus** | **Gene location in NCBI Reference Sequence** | **Promoter region** |
|---|---|---|---|---|---|
| 199598845 | ribosomal protein S14* | rps.J | LRH_03200 | NZ_ABWJ01000023.1:25789_25974 | 19083-19412 |
| 199598701 | nucleoid DNA-binding protein | *dnabp* | LRH_04048 | complement(NZ_ABWJ0 100002D.1:15543_15818) | c(15819-16051) |
| 199539325 | ribosomal protein S2 | *rpS21* | LRH_03358 | GOmplement(NZ_ABWJ01000036.1:5953_6129) | c(6130-6401) |
| 199598841 | ribosomal protein S17' | rpsJ | LRH_03180 | NZ_ABWJ01000023.1:24203_24466 | 19083-19412 |
| 199597121 | 50S ribosomal protein L29 | rplS | LRH_07296 | complement(NZ_ABWJ01000002.1:62543_62890) | c(63891-63094) |
| 199598859 | 50S ribosomal protein L17 | *map40* | LRH_03270 | NZ_ABWJ01000023.1:33024_33404 | 31990-32063 |
| 199598851 | ribosomal protein L15* | rpsJ | LRH_03230 | NZ ABWJ01000023.1:28112_28552 | 19083-19412 |
| 199598872 | 50S ribosomal protein L13 | rplM | LRH_03335 | NZ_ABWJ01000023.1:42964_43410 | 42698-42963 |
| 199587445 | phosphoglycerate mutase 1 | *pgm1* | LRH_13389 | NZ_ABWJ0100004.1:98387_99076 | 98118-98386 |
| 19P59B83B | ribosomal protein S3* | *rpsJ* | LRH_03165 | NZ_ABWJ01000023.1:22898_23560 | 19083-19412 |
| 199597503 | ribosomal protein S4 | *rpS4* | LRH_06231 | NZ_ABWJ01000005.1:20167_20778 | 19892-20166 |
| 199597272 | glyceraldehyde-3-phasphate dehydrogenase | *cggr* | LRH_05288 | NZ_ABWJ01000003.1:50087_51109 | 48208-49012 |

| | | | | | |
|---|---|---|---|---|---|
| * identical operon | | | | | |

**Table 2. Select genes and promoter regions identified in Streptococcus mutans (particularly S. mutans UA159).**

| **GI-number** | **Gene / Protein name** | **Locus** | **Gene location in NCBI Reference Sequence NC_004360 (version 1)** | **Promoter region** |
|---|---|---|---|---|
| 1350920 | 30S ribosomal protein S10 | SMU.2026c | complement(1893020_1893130) | c1893131-1893647 |
| 24379304 | 50S ribosomal protein L27 | SMU.849 | 797718_798011 | 796862-797035 |
| 24378669 | 30S ribosomal protein S15 | SMU.154 | 156212_156481 | 155470-156212 |
| 24378669 | 30S ribosomal protein S 16 | SMU.865 | 814687_814962 | 814285-814686 |
| 24379704 | 50S ribosomal protein L19 | SMU.1288 | complement(1214632_1214979) | c1214980-1215169 |
| 24380355 | 30S ribosomal protein S8 | SMU.2012 | complement(1887696_1888094) | c1888095-1888350 |
| 24380353 | 50S ribosomal protein L18 | SMU.2010 | complement(1886301_1886657) | c1886658-1886746 |
| 24378685 | 30S ribosomal protein S9 | SMU.170 | 170269_170661 | 169408-169768 |
| 24380352 | 30S ribosomal protein S5* | SMU.2009 | complement(1885788_1886282) | c1886658-1886746 |
| 15902260 | 50S ribosomal protein L17 | SMU.2000 | complement(1880033_1880419) | c1881377-1881421 |
| 24380345 | 30S ribosomal protein S13 | SMU.2003 | complement(1881823_1882188) | c1882569-1882686 |
| 24378855 | 30S ribosomal protein S7 | SMU.358 | 334520_334996 | 334997-335164 |
| 24380350 | 50S ribosomal protein L15 | SMU.2007 | complement(1834859_1885299) | c1885300-1885591 |
| 24380465 | 30S ribosomal protein S4 | SMU.2135c | complement(1999236_1999847) | c1999848-1999950 |
| 24380354 | 50S ribosomal protein L6 | SMU.2011 | complement(1886747_1887283) | c1887284-1887695 |
| 161486819 | 30S ribosomal protein S3** | SMU.2021 | complement(1890900_1891553) | c1892786-1893018 |
| 24379074 | phosphoglyceromutase | SMU.74 | 74927_75637 | 74855-74926 |
| 24380367 | 50S ribosomal protein L3** | SMU.2025 | complement(1892159_1892785) | c18927S8-1893019 |

| | | | | |
|---|---|---|---|---|
| *identical operon **identical operon | | | | |

The recombinant nucleic acid as taught herein, such as recombinant nucleic acid encoding the (preferably heterologous) expression product, may be comprised in a vector.

As used herein, "vector" refers to a nucleic acid molecule, typically DNA, to which nucleic acid fragments may be inserted and cloned, *i.e.,* propagated. Hence, a vector will typically contain one or more unique restriction sites, and may be capable of autonomous replication in a defined host or vehicle organism such that the cloned sequence is reproducible. Vectors may include, without limitation, plasmids, phagemids, bacteriophages, bacteriophage-derived vectors, PAC, BAC, linear nucleic acids, e.g., linear DNA, etc., as appropriate (see, e.g., (Sambrook et al., Molecular Cloning: A Laboratory Manual, 2nd ed., vol. 1-3, ed. Cold Spring Harbor Laboratory Press, Cold Spring Harbor, N.Y., 1989); (Ausubel et al., Current Protocols in Molecular Biology, ed. Ausubel et al., Greene Publishing and Wiley-Interscience, New York, 1992).

Factors of importance in selecting a particular vector, e.g., a plasmid, include *inter alia:* the ease with which recipient cells that contain the vector may be recognized and selected from those recipient cells which do not contain the vector; the number of copies of the vector which are desired in a particular host; and whether it is desirable to be able to "shuttle" the vector between host cells of different species. Preferred prokaryotic vectors include plasmids such as those capable of replication in *E. coli* (such as, for example, pBR322, ColE1, pSC101, pUC19, etc.). Such plasmids are describe in, e.g., (Sambrook et al., Molecular Cloning: A Laboratory Manual, 2nd ed., vol. 1-3, ed. Cold Spring Harbor Laboratory Press, Cold Spring Harbor, N.Y., 1989); (Ausubel et al., Current Protocols in Molecular Biology, ed. Ausubel et al., Greene Publishing and Wiley-Interscience, New York, 1992). Particularly preferred vectors may be those able to replicate in *E. coli* (or other Gram negative bacteria) as well as in another host cell of interest, such as in a Gram positive bacterium, a lactic acid bacterium, preferably *Lactobacillus.* Other preferred vectors may be those able to replicate and/or shuttle between one or more Gram positive bacteria but not in Gram negative bacteria. In a preferred embodiment, the vector is pT1NX as described by (Steidler et al., 1998 (Infect Immun 66(7): 3183-9) "Mucosal delivery of murine interleukin-2 (IL-2) and IL-6 by recombinant strains of Lactococcus lactis coexpressing antigen and cytokine", which is specifically incorporated by reference herein)

In a related aspect, the invention provides a method for delivery of a (preferably heterologous) expression product, such as polypeptide encoded by the one or more open reading frames comprised within the recombinant nucleic acid of the invention to human or animal in need thereof, comprising administering to the said human or animal a therapeutically effective amount of host cells (strain, microbe) transformed with the said nucleic acid and/or vector of the invention.

The animal may preferably be a mammal, such as, e.g., domestic animals, farm animals, zoo animals, sport animals, pet and experimental animals such as dogs, cats, guinea pigs, rabbits, rats, mice, horses, cattle, cows; primates such as apes, monkeys, orang-utans, and chimpanzees; canids such as dogs and wolves; felids such as cats, lions, and tigers; equids such as horses, donkeys, and zebras; food animals such as cows, pigs, and sheep; ungulates such as deer and giraffes; rodents such as mice, rats, hamsters and guinea pigs; and so on. Generally, the term "subject" or "patient" may be used interchangeably and particularly refer to animals, preferably warm-blooded animals, more preferably vertebrates, even more preferably mammals, still more preferably primates, and specifically includes human patients and non-human animals, mammals and primates. Preferred patients may be human subjects.

As used herein, the terms "treat" or "treatment" refer to both therapeutic treatment and prophylactic or preventative measures, wherein the object is to prevent or slow down (lessen) an undesired physiological change or disorder. A "human or animal in need of treatment" includes ones that would benefit from treatment of a given condition.

The term "therapeutically effective amount" refers to an amount of a therapeutic substance or composition effective to treat a disease or disorder in a subject, e.g., human or animal, i.e., to obtain a desired local or systemic effect and performance. By means of example, a therapeutically effective amount of bacteria may comprise at least 1 bacterium, or at least 10 bacteria, or at least 10² bacteria, or at least 10³ bacteria, or at least 10⁴ bacteria, or at least 10⁵ bacteria, or at least 10⁶ bacteria, or at least 10⁷ bacteria, or at least 10⁸ bacteria, or at least 10⁹, or at least 10¹⁰, or at least 10¹¹, or at least 10¹², or at least 10¹³, or at least 10¹⁴, or at least 10¹⁵, or more host cells, e.g., bacteria, e.g., in a single or repeated dose.

The host cells (strain, microbe) of the present invention may be administered alone or in combination with one or more active compounds. The latter can be administered before, after or simultaneously with the administration of the said host cells.

A number of prior art disclosures on the delivery of antigens and/or therapeutically active polypeptides exist, and it shall be appreciated that such disclosures may be further advantageously modified with the reduced-colonizing or non-colonizing strains of microbes as taught herein. By means of example and not limitation, bacterial delivery of trefoil peptides may be used to treat diseases of the alimentary canal (see, e.g., WO 01/02570), delivery of interleukins in particular IL-10 for treating colitis (e.g., see WO 00/23471), delivery of antigens as vaccines (e.g., WO 97/14806), delivery of GLP-2 and related analogs may be used to treat short bowel disease, Crohn's disease, osteoporosis and as adjuvant therapy during cancer chemotherapy, etc. Further therapeutic applications are envisioned using the promoters and host cells (strain, microbe) of the invention.

Further non-limiting examples of the types of diseases treatable in humans or animals by delivery of therapeutic polypeptides according to the invention include, but are not limited to, e.g., inflammatory bowel diseases including Crohn's disease and ulcerative colitis (treatable with, e.g., IL-Ira or IL-10 or trefoil peptides); autoimmune diseases, including but not limited to psoriasis, rheumatoid arthritis, lupus erythematosus (treatable with, e.g., IL-Ira orlL-10); neurological disorders including, but not limited to Alzheimer's disease, Parkinson's disease and amyotrophic lateral sclerosis (treatable with, e.g., brain devated neurotropic factor and ciliary neurotropic factor); cancer (treatable with, e.g., IL-1, colony stimulating factors or interferon- W); osteoporosis (treatable with, e.g., transforming growth factorf3); diabetes (treatable with, e.g., insulin); cardiovascular disease (treatable with, e.g., tissue plasminogen activator); atherosclerosis (treatable with, e.g., cytokines and cytokine antagonists); hemophilia (treatable with, e.g., clotting factors); degenerative liver disease (treatable with, e.g., hepatocyte growth factor or interferon a); pulmonary diseases such as cystic fibrosis (treatable with, e.g., alpha antitrypsin); obesity; pathogen infections, e.g., viral or bacterial infections (treatable with any number of the above-mentioned compositions or antigens); etc.

The skilled reader shall appreciate that the herein specifically recited diseases are only exemplary and their recitation is in no way intended to confine the use of the reagents provided by the invention, e.g., the reduced-colonizing or non-colonizing strains of microbes of the invention, to these particular diseases. Instead, a skilled reader understands that the reagents of the invention can be used to express in principle any expression products, preferably polypeptides, of interest, which may be of therapeutic relevance in not only the recited ones but also in various further diseases or conditions of humans and animals. Consequently, once a suitable expression product, preferably a polypeptide, e.g., an antigen and/or a non-vaccinogenic therapeutically active polypeptide, has been chosen or determined for a given ailment, a skilled person would be able to achieve its expression, isolation and/or delivery using the reagents of the invention.

The invention also contemplates treatment of diseases in other animals including dogs, horses, cats and birds. Diseases in dogs include but are not limited to canine distemper (paramyxovirus), canine hepatitis (adenovirus Cav-1), kennel cough or laryngotracheitis (adenovirus Cav-2), infectious canine enteritis (coronavirus) and haemorrhagic enteritis (parvovirus).

Diseases in cats include but are not limited to viral rhinotracheitis (herpesvirus), feline caliciviral disease (calicivirus), feline infectious peritonitis (parvovirus) and feline leukaemia (feline leukaemia virus). Other viral diseases in horses and birds are also contemplated as being treatable using the methods and compositions of the invention. To this purpose, the use of microorganisms expressing recombinant interferons will be particularly preferred.

In a further aspect, the invention thus also provides a pharmaceutical composition comprising the host cell (strain, microbe) as taught herein, optionally transformed with the nucleic acid and/or the vector for a (preferably heterologous) expression product.

Preferably, such formulation comprise a therapeutically effective amount of the host cells of the invention and a pharmaceutically acceptable carrier, *i.e.,* one or more pharmaceutically acceptable carrier substances and/or additives, e.g., buffers, carriers, excipients, stabilisers, etc.

The term "pharmaceutically acceptable" as used herein is consistent with the art and means compatible with the other ingredients of a pharmaceutical composition and not deleterious to the recipient thereof.

The recombinant host cells of the invention can be suspended in a pharmaceutical formulation for administration to the human or animal having the disease to be treated. Such pharmaceutical formulations include but are not limited to live host cells and a medium suitable for administration. The recombinant host cells may be lyophilized in the presence of common excipients such as lactose, other sugars, alkaline and/or alkali earth stearate, carbonate and/or sulphate (for example, magnesium stearate, sodium carbonate and sodium sulphate), kaolin, silica, flavorants and aromas.

Host cells so-lyophilized may be prepared in the form of capsules, tablets, granulates and powders, each of which may be administered by the oral route.

Alternatively, some recombinant bacteria may be prepared as aqueous suspensions in suitable media, or lyophilized bacteria may be suspended in a suitable medium just prior to use, such medium including the excipients referred to herein and other excipients such as glucose, glycine and sodium saccharinate.

For oral administration, gastroresistant oral dosage forms may be formulated, which dosage forms may also include compounds providing controlled release of the host cells and thereby provide controlled release of the desired protein encoded therein. For example, the oral dosage form (including tablets, pellets, granulates, powders) may be coated with a thin layer of excipient (usually polymers, cellulosic derivatives and/or lipophilic materials) that resists dissolution or disruption in the stomach, but not in the intestine, thereby allowing transit through the stomach in favour of disintegration, dissolution and absorption in the intestine.

The oral dosage form may be designed to allow slow release of the host cells and of the recombinant protein thereof, for instance as controlled release, sustained release, prolonged release, sustained action tablets or capsules. These dosage forms usually contain conventional and well known excipients, such as lipophilic, polymeric, cellulosic, insoluble, swellable excipients. Controlled release formulations may also be used for any other delivery sites including intestinal, colon, bioadhesion or sublingual delivery (i.e., dental mucosal delivery) and bronchial delivery. When the compositions of the invention are to be administered rectally or vaginally, pharmaceutical formulations may include suppositories and creams. In this instance, the host cells are suspended in a mixture of common excipients also including lipids. Each of the aforementioned formulations are well known in the art and are described, for example, in the following references: Hansel et al. (1990, Pharmaceutical dosage forms and drug delivery systems, 5th edition, William and Wilkins); Chien 1992, Novel drug delivery system, 2nd edition, M. Dekker); Prescott et al. (1989, Novel drug delivery, J. Wiley & Sons); Cazzaniga et al., (1994, Oral delayed release system for colonic specific delivery, Int. J. Pharm.i08:7').

Preferably, an enema formulation may be used for rectal administration. The term "enema" is used to cover liquid preparations intended for rectal use. The enema may be usually supplied in single-dose containers and contains one or more active substances dissolved or dispersed in water, glycerol or macrogols or other suitable solvents.

Thus, according the invention, in a preferred embodiment, reduced-colonizing or non-colonizing strains of microbes as taught herein, such as recombinant host cells encoding a desired gene may be administered to the animal or human via mucosal, e.g., an oral, nasal, rectal, vaginal or bronchial route by any one of the state-of-the art formulations applicable to the specific route. Dosages of host cells for administration will vary depending upon any number of factors including the type of bacteria and the gene encoded thereby, the type and severity of the disease to be treated and the route of administration to be used.

Thus, precise dosages cannot be defined for each and every embodiment of the invention, but will be readily apparent to those skilled in the art once armed with the present invention. The dosage could be anyhow determined on a case by case way by measuring the serum level concentrations of the recombinant protein after administration of predetermined numbers of cells, using well known methods, such as those known as ELISA or Biacore (See examples). The analysis of the kinetic profile and half life of the delivered recombinant protein provides sufficient information to allow the determination of an effective dosage range for the transformed host cells.

In an embodiment, when the host cells (strain, microbe) express an antigen, the invention may thus also provide a vaccine. Preferably, the antigen may be capable of eliciting an immune response in and used as a vaccine in a human or animal.

The term "vaccine" identifies a pharmaceutically acceptable composition that, when administered in an effective amount to an animal or human subject, is capable of inducing antibodies to an immunogen comprised in the vaccine and/or elicits protective immunity in the subject.

The vaccine of the invention would comprise the host cells (strain, microbe) as taught herein, optionally transformed with the nucleic acids or vectors encoding the antigen and further optionally an excipient. Such vaccines may also comprise an adjuvant, i.e., a compound or composition that enhances the immune response to an antigen. Adjuvants include, but are not limited to, complete Freund's adjuvant, incomplete Freund's adjuvant, saponin, mineral gels such as aluminum hydroxide, surface active substances such as lysolecithin, pluronic polyols, polyanions, peptides, oil or hydrocarbon emulsions, and potentially useful pharmaceutically acceptable human adjuvants such as BCG (bacille Calmetle-Guerin) and *Corynebacterium parvum.*

In an embodiment, when the host cells (strain, microbe) express a non-vaccinogenic prophylactically and/or therapeutically active peptide, polypeptide or protein, the invention may thus also provide a tool to deliver the same to a subject. The host cells may be administered as a suitable pharmaceutical formulation or composition. Hence, the formulation or composition would typically comprise the host cells (strain, microbe) as taught herein, optionally transformed with the nucleic acids or vectors encoding the non-vaccinogenic prophylactically and/or therapeutically active peptide, polypeptide or protein and further optionally a pharmaceutically acceptable excipient.

The invention is further illustrated with examples that are not to be considered limiting.

### Examples

### Example 1

### Construction of thyA deficient mutant strains

From *Streptococcus mutans, Lactobacillus acidophilus, Lactobacillus plantarum and Lactobacillus salivarius WCFS1,* we have cloned out 1000 bp regions flanking the thyA gene. The DNA sequence information for the genetic engineering of any *Streptococcus* and *Lactobacillus* strain in a way as described below are available from public sources (Table 1).

The strategy to generate thyA deficient strains employs double homologous recombination in the areas 1000 bp at the 5' end (SEQ ID N 1-4) and 1000 bp at the 3' end (SEQ ID N 5-8) of thyA, the "thyA target regions".

We have cloned these flanking DNA fragments in a derivative of pORI19 (Law et al., 1995 (J Bacteriol 177(24): 7011-8) a conditionally replication defective plasmid. Transformation of the plasmids in any of the *Streptococcus* and *Lactobacillus* strain only transfers the erythromycin resistance when a first homologous recombination occurs at either the 5' 1000 bp or at the 3' 1000 bp of the thyA target. A second homologous recombination at the 3' 1000 bp or at the 5' 1000 bp of the thyA target removes the thyA gene and yield the desired strain.

As an option, in between both 5' and 3' target regions additional genes are inserted, which will replace thyA in the thyA deficient strain.

### Evaluation of colonization

Wild type Streptococcus mutans, Lactobacillus acidophilus, Lactobacillus plantarum and Lactobacillus salivarius bacteria were transformed with a plasmids encoding a chloramphenicol (Cm) selection marker. Their respective thyA-deficient mutants were transformed with a plasmid encoding an erythromycin (Em) selection marker (the above: or vice versa).

Suspensions of the Cm+ wild type bacteria and of the Em+ thyA mutant bacteria were mixed and inoculated in the oral cavity, in the stomach through oral gavage or in the vagina. Daily samples from the oral cavity, stool and vagina were plated on either Cm or Em selective agar plates.

The relative presence of the Cm+ wild type bacteria and of the Em+ thyA mutant bacteria were determined by plate count and have shown a significant reduction of colonization of the Em+ thyA mutant bacteria in comparison to the Cm+ wild type bacteria.

**Table 1: Public sources for sequence information required for the construction of thyA deficient mutants of the indicated organisms.**

| Organism | Genome sequence | thyA gene | 5'thyA target | 3'thyA target |
|---|---|---|---|---|
| Streptococcus mutans UA 159 | GenBank AE014133 Refseq NC 004350 | GenelD 1028296 | SEQ ID N 1 | SEQ ID N 5 |
| [1] | | | | |
| Lactobacillus acidophilus NCFM | GenBank CP000033 Refseq NC 006841 | GenelD 3251830 | SEQ ID N 2 | SEQ ID N 6 |
| [2] | | | | |
| Lactobacillus plantarum WCFS1 | GenBank AL935263 Refseq NC 004567 | GenelD 1064048 | SEQ ID N 3 | SEQ ID N 7 |
| [3] | | | | |
| Lactobacillus salivarius UCC118 | GenBank CP000233 Refseq NC 007929 | GenelD 3976937 | SEQ ID N 4 | SEQ ID N 8 |
| [4] | | | | |

| | | | | |
|---|---|---|---|---|
| [1] (Ajdic et al., 2002 (PNAS 99(22): 14434-9) [2] (Altermann et al., 2005 (PNAS 102(11): 3906-12) [3] (Kleerebezem et al., 2003 (PNAS 100(4): 1990-5) [4] (Claesson et al., 2006 (PNAS 103(17): 6718-23) | | | | |

**SEQ ID N 1** 
CAAAATTCACCTTAAGTTTTTCCTGCAAATCAGATGGACTTGCTTATCCATGATTTTGT
TAAAGCCTTTAAGAGCTAGTTTAGGAAAAGGAAGATGAAAGCAAAAAGCTGCAAAATCAG
TTAAAGAAACATCAAAACGTTTTTGATATTCTGCCCAAGTCGTTTTCAGCATATCCAAAT
ATTGTTTGGTAGAATACATACCATTAACATAAGGTGTTGTTGTATAATTTGGCCGCCAAA
AATCCATAATATCACGTGTCTGAGCTAAGGTCTCATCATGAAGAATAAGAATACGGGGAT
CTTTTTTGACCAGCATGGCAATGCTGCCGGCTCCTTGAGTAGATTCTCCGGGAGTTCCA
ATTCCGTATTTGGCAATATCGCTAGCAAGAACAAGCACACGCGTGTCAGGATGTTTTTCA
ACATGCAGTTTAGCATAGTTGAGAGCTGCAGTAGCACTGTAGCATGCCTCTTTCATCTCA
AAGCTGCGCGCAAAAGGCTGAATACCTAATAAGGAATGCACATAAACAGCTCCAGCCTT
ACTTTGATCAACGCTGGATTCGGTAGCTAAAATGACCATATCAATTTTTTCTTTATCCTCT
GCAGTCAGAATTTCATTGGCTGATCCGGCTGCTAGGGTGACAACGTCGTCGGTAATAGG
AGCGATGCTAAGAGCATTCAGCAAAAGTCCTTTACTGAATTTTTGGGGATCTTCGCCACG
GGCCTCAGCTAAATCTTTCATATTCAGGACATATTGGCTGCTTGTAAAACCAATTTTATCA
ATCCCAATTCTCATAAGTCAATCCTTCTTTTCTTTTTTATCTGATTTCTTTTTTGTAAAAT
GTCATAGTTATTTTATCACAATTTAAACTAAAGGTAATGAATAAACTTTCAAGGATTCAA
TTTTTGGGAGAATCTGTCAGATCAGACGAAACCTATTTTTTCTGCTATAATATCTAAAAG
AAGTTTAAATTATTTTCTTAGAATCATAGAAGGAGAAGCA
**SEQ ID N 5** 
AAATGACGAGCCAATAATTATGGCTCTTTTTATAAAAACCAATATATGTCTTGACACA
TGTAAAAAACGTTGTATAATGATTTCAAACATTAATTAAAAAGGTGGAGCAAAACGGTGT
 
CTAGAAAAGCAAGCAATATCTGGATAATTTTAAATGGCTGTGGCAAGAAGGTAAAAAAT
TAGGCTTTTGGGGGATATTACAAGCGCTATGGAGGATTTGGTTAAAAATAGGAGCCTG
TCGCAGTGGCTATACCTTTTAGCTCTTAGTTTTCCAACACTGGTTTTAGAATTTATCGGTG
GAACACGGCACATTGCTGGTTTTGCGGCAGCACTGACAGGAATTCTATGTGTTATCTTTG
TTGCTGAGGGGCGTATCAGTAATTATTTCATCGGTTTTATTCATGAAATGCTCTATCTTT
ATCTCAGTTTTGAAAATATGTATTATGGTGAAGTTTTAACAACCTTATTTTTCACTGTCA
TGCAATTTGTCGGTGCTTATTATTGGTTGATTGGACATCGTGAAGGACAGGGAAAGAAAG
TTGAAGTAAAGGATGTTAAATCACGAAAACTGACACCCCTAGGCTGGTTGAAATCACTTG
GAATTACAATTATTGTTTGGTTAGTTTTTGGCTTTATTTATCGATCAATCGGTTCGCATC
GCCCTTTTTGGGATAGTTCAACAGATGGTACCAATTGGAGCGGTCAGTTTCTTCAAACAG
GCATGTACAGTGAACAATGGCTCTTTTGGATTGCGACCAATGTTCTCAGCATTTTTCTTT
GGTGGGGTGCAGAACCACATGTTATGCTCATGTATATTATTTATATGATTAATAGTATTG
TTGGTTGGGTAAAATGGGAACGTGATCTTAGAATAACTCAAGAACAGTTAGCTTAAATAA
GGAGTTTAAATACAATAGACAAAATGCATTTGAGTTTTTCAATGCATTTTTTTAGAAA
TTTGATAAAATAAGAAAAGTATGAACAATAAGCGAGAAAA
**SEQ ID N 2** 
ATTTTCTTTGGTGTAGGCAACGGTGGAAAGCCAATTGGTTTTAGCAATTTATGGGCTCAT
GGCGGTTTCTTTACTGGCGGAGTAAAAGGCTTTTTCTTCTCGATGTCAATTATTGTTGGT
TCATATGAAGGAATTGAGTTGCTAGGCATTTCGGCAGGCGAGGTGGCTAACCCGCAAAA
AGCAATTGTAAAGAGTGTCAAGTCTGTTCTTTTGAGAATTTTAATTTCTACGTTGGTGCA
ATTTTTGTGATTGTAACTATTTATCCGTGGAATAAGTTAAGTAGCTTAGGTTCTCCTTTT
GTAACTACCTTTGCTAAGGTAGGAATCACTGCTGCTGCTTCGATTATTAACTTCGTTGTA
TTAACTGCAGCATTGTCAGGAGCTAACTCGGGGATTTATTCATCAAGTAGAATGCTATTT
AAATTGTCTCATGATAATGAAGCACCTAGTGTATTTAAGCATATTTCTAAGAGAATTGTG
CCTGATCGCGCCATTATGGGGATTTCTGGTGGGATTTTTATTGGGTTTATTTTAAATATA
ATTGCATCTCAATTTAATCATTCGGCATCAGATTTATTTGTGATTGTCTTCAGTTCATCA
GTTTTACCAGGAATGATTCCATGGTTTGTAATTCTATTGGCTGAGTTAAGATTTAGAAGA
CATAATCAAGATATGATGAAAGATCACCCGTTCAAATTGCCGTTATATCCATTTTCTAAT
TACTTCGCATTTTTAATGCTGTTAGTAATTGTTATCTTTATGTTTATTAATCCAGATACT
AGAATTTCAGTAATTACCGGAGCATTGGTATTAATTGTGGCTACAATTGTTTATTTAGTT
AGACATAAAGATGAATTTAGTAAAAATAATTAATGATTAAGAAGTCTTGAAATTTCAGGG
CTTTTTATTTTTACCAAATACTAAATATTGATACTTGCATTATCAAAAATACTAGATCTA
 TGGTATCTTAAAAAGAAATGATACTTAAAGGGTGAGATAA
**SEQ ID N 3** 
TTCGCTCGGTCAGCAGCGTTTGGGTAAGAAAGTGATCGAGTTAAAGGACGCGAGCTTGC
AGTTTGATCGGCAAACCATCCTGGATCACTTTTCGATGTTGATCCAAGCCAATGACCGCA
TCGGTATCACTGGAATCAACGGTGCCGGAAATCTAGTTTATTGAATGTGATTGCGGGT
CGACTGCCACTTGATAGTGGACCGTGACGATCGGTGAGACCGTCAAGATGGCCTATTA
TACTCAACAGACCGAACCGATTCCGGGCGACAAGCGCATCATTAATTACTTGCAAGATG
TCGGTGAGACGGTCTTGAATAAGCAGGGCGAGCATGTTTCAGTGACTGAGTTACTAGAA
GAGTTTCTGTTCCCACGTTCGATGCACGGCACGCTGATTCGCAAGTTATCTGGGGGCGA
ACAGCGCCGGCTATACTTACTGAAGTTGTTGATGCAACAGCCGAACGTCCTCTTATTGG
ACGAACCGACTAATGATTTGGATATTGGTACGTTAACGGTGTTGGAGAACTACCTCGATG
ATTTTGCCGGGACCGTCATCACTGTTTCCCATGACCGCTATTTTTGGACAAGGTCGGCA
CGAAACTACTGATTTTTGATGGTCAGGGGCATATTGAACGCTATTCTGGCCGTTTCTCCA
GTTATTTGAAGGATCAAAAAGACGCGGCCAAGCCCGCAGCCAAGGCCACTGCGACAAA
AACTACGGCTAAGCCGGTCACGGATGACTCGACCGCACCATTGGCCAAGAAAGCCAAA
GTCAAGCTGACTTACGCGGAACAGCTCGAATACGACAAGATCGAAGGGGTTATCGAGCA
ATTGGATAGCCATAAGAGTGAGATCGAAGCGGCGATGGCTGCCAATGCCAGTGATTATG
GCAAGTTGGCTGATTTGCAAAAAGAATTGACTAAGACGGAACAAACGATTGATGAGAAG
ATGGATCGCTGGGACTACCTCAGCCAGTATGCAGAAGCCTGAAAGGATGATCGC
**SEQ ID N 6** 
GGTGAAGTAATGATTGAATATGTTTGGGCAGAAGATAAAGAAAAAAATATTGGCTTGAAT
GGACATTTACCATGGTATTTGCCGGCTGATATGAAGCATTTTAAAGAAGTAACAATTAAT
CATCCAATAATTATGGGAAGAAAAACATTTGAAAGTTTTCCTAATTTGTTACCTAAAAGA
AAACATATTGTTTTAACTCATAATGAAGAGCTAAAAAATAAATATCAAAATAATGATCAA
GTGACTATTTTACCCACAGTTGAAGATTTACATAATTTTGTGGCAGAACATCAAGATGAG
CGGATGTGTGCAATTGGTGGAGTGTCGATTTTTAACGCTTTAATGGACCAAGTAGAAGTA
TTAGAAAAAACGGAGATAGATGCGATTTTTGAAGCAGATACTAAAATGCCTGAAATTGAT
TATAGCCGTTTTAATTTAGTAGCTAAGAAGCATTATGAGCCGGATGAGAAAAATAAGTAT
CCATATACTTTTTTAACTTATAAATTAAAGTAAAAAAAGCCCGCAATTTGCGAGCTTTTT
TGAATCTTAGGTATAATATTAATAAATATTAAGTTTCTTAGGTAATAATATATGAATGAA
GAAAAAACAAATTTAAATACGGGCCTGACAGCTGCACAAGTTAAGCAAAAAATTGAAGCT
 
GGTGAAATTAATAAGGCCGTTGATGATCAATTTAAAACAAATAAACAAATTATTGCAGAA
AATTTATTTACTTACTTTAACTTGATTTTCTTAGTACTATCATTACTTTTGATTTTTGTT
GGCGCATATAAGGATTTAACTTTTTTGCCAGTCATTGTTTTAAATACTGTAATTGGAATT
GTGCAAGAAATTCGTGCTAAAAAAATATTGAACAAGTTAAACGTAATGAATGCTACAGAT
ATTGGGGCTTTGCGAGATGGAAAAGAAGTACAAGTACCAATAGAAGAGTTAGTTAAAGG
CGATATTGTTTTATTAAAAACAGGCGATCAGATTCCAGCAG
**SEQ ID N 7** 
GAGGCCGCAATGATTGCATTGATATGGGCAGAAGATCAAAATGGCCTGATTGGCAACCA
GGGCCAGCTTCCCTGGCATTTACCAGCAGACATGCAGCGGTTCAAAGCGCTGACGACC
GGACACCATGTCGTGATGGGGCGCAAGACTTTTGCGGGCTTTAAACGCCCGCTCCCAC
GGCGGACCAATTGGGTGCTCTCACGTCAGTCTGATTTAAAGTTGCCACCGGAAGTCCAT
CAACTAGCAGATGTGGCGGCGATCCAAACACTCGCGGCCGCCCATCCGGATGAACCGA
TTTTTGTCATTGGTGGTGCGGTGGTGTTTGAAGCCGTGTTACCGGTGGCCGATTATTTAT
ATCGGACGCGTATCAACGCAAGGTTTGATGGTGATACTTGGATGCCGGCCGTGGATTAC
ACGCAGTGGCAGCTGGTGAGCCAACAAATTGGGACGGTGGATGAGAAAAATCAATATCC
GTACGAATTTGATGATTTCCGCCGTCGTTAATTCAGTGGCAACCGTGAAACTCAGGCAG
ATTTCTGCTATACTGAAAGCATTAATTTTATTGTAACGATCGTTTAAACGTTACAAGTATAA
GAAAGCGTGGCAAAGTATGACGAAAATCAAGATTGTGACCGATTCTTCGGCAAATTTGAC
GGATGCCGAGGTAAAAAATACGATATTACCGTGATTCCGTTGACTGTCATGATCGATGG
CACCATTTACGTGGAAGATGAAACGATTACGCGTGAAGAATTTATTGACAAGATGGCAAC
GGCTAAATCTTTACCAAAGACGAGCCAACCGGCACTCGGAACGTTCATTGAAACGTTTG
ATAAGTTAGGTGCGGATGGCGCGAGTGTGATTTGTATCAATATGCTCGAAGCCATTTCA
GGAACAGTGCACACGGCGGAACAAGCGGCTTCGATTACGAAGACCGATGTCACCGTGA
TCGACGCTCGGACGACCGACCGTGCGATGGCCTTTCAAGTTTTGACTGCTGCT
**SEQ ID N 4** 
TTGATATTAATTTAGGACAACAAAGATTGGGAAAGAAAGTATTAGAATTAAAAGATGCAA
GTTTAACAATTGGAAATCATAAAATAATTGAAGATTTCAATTTGCTAATCCAAGCTGGAG
ACAGAATCGGTATTACAGGCGTTAATGGTGCCGGAAATCTAGTTTTTAAATGCTTTAT
CAGGTGAATTGCCATTAGATTCAGGTATTTTGACAATCGGTGAAACTGTAAAGATGGCTT
ATTATCGACAACAGACGGAAGAGATTCCTGAAGATAAAAGAATTATTAGTTATTTAAACG
AAGTTGGTCAAAATATTGTAAATAAAGATGGTGAGCGAATTAGCACAACTCAATTATTGG
 
AACAATTTCTATTTCCAAGATTTATGCATGGAACATTGATTCGCAAACTCTCTGGCGGTG
AAAAAAGACGCTTGTATCTTTTGAAATTATTAATGTCTCAACCTAATGTTTTATTATTGG
ATGAACCAACTAACGATTTAGATATAGGAACATTAACAGTTTTGGAAGATTATTTAGATA
ACTTTAACGGAACTGTTATTACCGTATCACATGACAGATATTTTCTTGATAAGGTAGCTG
ATTCATTATATATCTTTGAAGGAAATGCAAAAATCAAGCATTATGTCGGAATGTTTACAG
ATTATCTGAAGAATGCTGAGAATGAAGCAGTTAAGACTAAAAAACAAGTATCTGCAACAA
AAGTAGAAAAGACAGATAGTGCAGAAGACAAAGTTAAAAAGAAAACCAAGTTAACTTACG
CTGAAAAGATGGAATATGAAAAATTAGAATCAGAAATCGATAAGCTTGAAAATGATAAAG
CAAGTTTAGAAGAAGAAATGCAACATGTTGATGGGGCTGATTATACTAAATTAGCAAGTT
TACAGCAACAAATTGATGAATTAGATGAAGATATTATGGAAAAAGTTCAACGTTGGGACG
AACTTAGTCAGTATGTTGATTAATAGGAAGGAGAACACCG
**SEQ ID N 8**

TCTGTGAGGTGTGCACTATGATTTCATTTGTATGGGCAGAAGATCAAAAACATCAAATAG
GATATAAAGGACATTTACCTTGGAGACTTCCAGCCGATTTGGCTCATTTAAAGAAGTTA
CAATGGGTCATCCAATGGTAATGGGGAAGAAAACGTTTGATAGTTTTCCTGGTTTATTAC
CAAGACGTCAACATATAGTGTTAACCCATGATACTAACTTAGAAGAAAAGTATAAAGATA
ATCCCCAAGTAGAAATTATGAATTCTATTGATGAATTAACTTCTTGGTTAGATGAAAATC
AGTTTCAAGAAGTTAGCGTTATTGGTGGAGCGATGTTATTCAACTTACTTCTTAATAAAG
TAGATAAATTATATAAGACTGAAATACTAAGTGAGTTTAATGGCGATACGGTTATGCCTA
CTATTAATTATGATGAATTTAAACTTGTTTCAAAAAAAATCGGTAAGGTAGATGAAAAAA
ACAAGTATCCTTATGTATTTTTGGAATACGAAAGAAAATAATTTCTAATAAAAATAATGA
AAGATGAGTAAGACAATTTTGTAGTACTCATCTTTTTTTACATTTTTAAGGCTTAAATGT
TGACTTCAAGTTAACTTGAAGTTTTATTATAGATATGTAAGTTCATATAGGGTTGATATT
AAAAACTCTAAATTTTTAAGAGAAGGATGTGCTAAATATGCAAAAATTATCAAGTTTAAC
ATCGCCATTAGTTCTAGCGAATGGTGTTGAAATTCCAGGACTAGGCTATGGTACTTACCA
AACTCCACCTGAAGATACATACAAAGTAGTATCAGAAGCATTATCAATCGGTTATCGCCA
CATTGACACAGCTGCATTATATGGCAATGAAAGTGGTGTAGGTAAGGCTGTTAAAGATAG
TGGGTTGAAGCGTGAAGAAGTATTTATTACTAGTAAGTTATGGAATACTGAACGTGGATA
CGACAAAGCAATTGCTTCCTTTAATAAGACTTTAGAAAAT

### Example 2: Effect of thyA deficiency on the colonizing capacity of Streptococcus mutans

*Streptococcus mutans* is a Gram positive, facultative anaerobic, lactic acid producing bacterium. It is a species that colonizes the human as well as hamster dental surface. As such it is an interesting candidate to serve as a host organism for delivery of therapeutic proteins to the oral mucosa. Hence, the effect of thyA deficiency on the colonizing capacity of *S. mutans* was investigated herein. We have used wild type *S. mutans* strain Clarke 1924 AL (LMG 14558; ATCC 25175; NCTC 10449) for all our experimentation.

### Example 2A: Construction of thyA- uidA+ Streptococcus mutans

Through double homologous recombination we have replaced thyA of *S. mutans* strain Clarke 1924 AL, with the uidA gene, encoding *E. coli* β-glucuronidase (GUS) **(****Figure 1****,** **Figure 2****).** The construction strategy uses a conditionally-replicative plasmid, pAGX0725, which was introduced in *Streptococcus mutans* Clarke 1924 AL under non-permissive circumstances. The plasmid pAGX0725 carries the uidA gene in-between 1kb regions cloned from chromosomal DNA upstream (thyA 5') or downstream (thyA 3') of thyA. Plating on erythromycin containing solid agar plates only allows for growth of those colonies in which a homologous recombination at either the thyA 5' or thyA 3' region had occurred. In this example, the recombination had taken place at both regions within the generations that led to growth of the first colonies, as was verified by PCR. The resulting *S. mutans* strain was named sAGX0108. The structure of the genetically modified region was verified by PCR and shows the presence of recombination events at thyA 5' and thyA 3', presence of the uidA gene as well as absence of thyA, all of which are in contrast with the structure of this region in *S. mutans* Clarke 1924 AL **(****Figure 2****).**
Genetically *S. mutans* sAGX0108 is thyA- uidA+. This phenotypically leads to strict growth dependence of *S. mutans* sAGX0108 for the addition of thymidine to the growth medium **(****Figure** 3).
Further, *S. mutans* sAGX0108 shows β-glucuronidase activity, as can be observed by plating on agar plates (BHI+T) containing 5-bromo-4-chloro-3-indolyl-beta-D-glucuronic acid, cyclohexylammonium salt (X-Gluc). Wild type *S. mutans* Clarke 1924 AL remain white and S. *mutans* sAGX0108 colonies show a clear, intense blue precipitate.

### Example 2B: In vivo evaluation of the colonizing capacity of thyA+ and thyA- S. mutans

We investigated the colonizing capacity of thyA+ and thyA- *S. mutans* in hamsters. This was done by inoculating thyA+ and thyA- *S. mutans* in the cheek pouch of hamsters. For these *in vivo* colonization studies, a method to discriminate inoculated thyA+ and thyA- *S. mutans* from resident microflora in the hamster oral cavity is required. Because mixtures of thyA+ and thyA- *S. mutans* are inoculated, this method must also allow mutual discrimination of both strains.

We transformed wild type *S. mutans* Clarke 1924 AL with the plasmid pILPOL which carries an erythromycin resistance marker. *S. mutans* Clarke 1924 AL pILPOL can therefore be grown on erythromycin containing BHI solid agar plates. *S. mutans* sAGX0108 can be discriminated from the background on the basis of β-glucuronidase activity, which leads to blue staining following plating on 5-bromo-4-chloro-3-indolyl-beta-D-glucuronic acid (X-gluc) and thymidine (T) containing BHI solid agar plates. X-gluc is a substrate for β-glucuronidase which cleaves X-Gluc to produce colorless glucuronic acid and an intense blue precipitate of chloro-bromoindigo. Wild type *S. mutans* Clarke 1924 AL do not grow on erythromycin containing BHI solid agar plates and colonies remain white when plated on BHI solid agar plates containing X-gluc and thymidine.

Hence, when plating the respective strains, the following discriminatory properties of *S. mutans* strains Clarke 1924 AL pILPOL and sAGX0108 have been observed: *S. mutans* Clarke 1924 AL pILPOL shows normal growth on BHI solid agar plates containing erythromycin; *S. mutans* sAGX0108 shows intense blue staining of colonies when plated on BHI solid agar plates containing X-gluc and thymidine; *S. mutans* Clarke 1924 AL shows no growth on BHI solid agar plates containing erythromycin; and *S. mutans* Clarke 1924 AL shows no blue staining of colonies when plated on BHI solid agar plates containing X-gluc and thymidine.

Using the same approach, *S. mutans* Clarke 1924 AL pILPOL and *S. mutans* sAGX0108 can be mutually discriminated. While *S. mutans* sAGX0108 shows intense blue colonies, S. *mutans* Clarke 1924 AL pILPOL colonies remain white when plated on X-gluc and thymidine containing BHI solid agar plates. In contrast to *S. mutans* Clarke 1924 AL pILPOL, *S. mutans* sAGX0108 shows no growth when plated on BHI solid agar plates containing erythromycin. 50:50 mixtures of both strains plated on X-gluc and thymidine containing BHI solid agar plates therefore show equal numbers of blue and white colonies (representing *S. mutans* sAGX0108 and *S. mutans* Clarke 1924 AL pILPOL respectively) while plating of an identical amount of this mixture on BHI solid agar plates containing erythromycin renders a colony number which is half of the above number of blue and white colonies (representing *S. mutans* Clarke 1924 AL pILPOL only). Both *S. mutans* Clarke 1924 AL pILPOL and *S. mutans* sAGX0108 can in this way clearly be discriminated from background microflora.

This is demonstrated in the following test. *S. mutans* Clarke 1924 AL pILPOL and *S. mutans* sAGX0108 were grown overnight to saturation and cultures were diluted 10⁶ fold. From this diluted suspension, single strains and 50:50 mixtures of the two strains were plated on either BHI+E or BHI+T+X-Gluc solid agar plates, as indicated. Single strains were plated as 100µl volumes while for mixtures, 100µl of the 50:50 mixture was plated. Colony numbers are shown in the following Table 2.

**Table 2. Mutual discrimination of S. mutans Clarke 1924 AL pILPOL and S. mutans sAGX0108 (1: S. mutans Clarke 1924 AL pILPOL; 2: S. mutans sAGX0108; 1+2: S. mutans Clarke 1924 AL pILPOL + S. mutans sAGX0108).**

| | 1 | 2 | 1+2 |
|---|---|---|---|
| BHI + E | 30 colonies | 0 colonies | 44 colonies |
| BHI+T+C-Gluc | white: 79 colonies | blue: 134 colonies | white: 55 colonies |
| | | | blue: 44 colonies |

*S. mutans* Clarke 1924 AL pILPOL shows clear growth on BHI+E solid agar plates but does not show blue staining on BHI+T+X-Gluc solid agar plates. *S. mutans* sAGX0108 shows no growth on BHI+E solid agar plates but shows clear blue staining on BHI+T+X-Gluc solid agar plates. This enables the clear discrimination of a mixture of both strains.

We recorded the fate over time of thyA- *S. mutans* sAGX0108 and thyA+ *S. mutans* Clarke 1924 AL pILPOL following inoculation of a mixture of both strains in the cheek pouch of hamsters **(****Figure 4****).** Despite the fact that the inoculum contained over 4x more of the thyA-strain, samples from the dental surface as well as cheek pouch showed reduced colonizing capacity of the thyA- strain. For both strains, in both cheek pouch as well as on dental surface, an initial increase in concentration was observed between day 0 and day 1. Subsequently, concentration of both strains decreased (**Figure 4****,** Panels A and C) but this reduction was substantially faster for thyA- *S. mutans* sAGX0108 (**Figure 4****,** Panels B and D). Moreover, no thyA- *S. mutans* sAGX0108 could be detected in the cheek pouch on day 7 and 10 while thyA+ *S. mutans* Clarke 1924 AL pILPOL were still present at those time points.

The findings of the above experiment were confirmed in a separate experiment (example 2C).

### Example 2C: In vivo evaluation of the colonizing capacity of thyA+ and thyA- S. mutans

*S. mutans* Clarke 1924 AL pILPOL and *S. mutans* sAGX0108 were transformed with a plasmid conveying chloramphenicol resistance. This yielded strains *S. mutans* Clarke 1924 AL pILPOL Cm+ and *S. mutans* sAGX0108 Cm+. We however found that chlorampheincol resistance was unreliably inherited over generations when strains were grown at 33°C or higher, which is not compatible with experimentation in vivo in hamsters.
For this reason we used the same approach as described in Example 2B to follow the fate of both strains. Again, *S. mutans* Clarke 1924 AL pILPOL Cm+ and *S. mutans* sAGX0108 Cm+ could be mutually discriminated and both *S. mutans* Clarke 1924 AL pILPOL Cm+ and S. *mutans* sAGX0108 Cm+ can in this way clearly be discriminated from background microflora. We recorded the fate over time of thyA- *S. mutans* sAGX0108 Cm+ and thyA+ *S. mutans* Clarke 1924 AL pILPOL Cm+ following inoculation of a mixture of both strains in the cheek pouch of hamsters **(****Figure 5****).** Despite the fact that the inoculum contained over 2x more of the thyA- strain, samples from the dental surface as well as cheek pouch showed reduced colonizing capacity of the thyA- strain.
In the cheek pouch, both strains were stably maintained over 1 day. As from day 3 thyA- S. *mutans* sAGX0108 Cm+ could no longer be detected while thyA+ *S. mutans* Clarke 1924 AL pILPOL Cm+ were still present at all time points (**Figure 5****,** Panels A and B)
On dental surface, the presence of both strains showed an initial decrease. As from day 3 no thyA- *S. mutans* sAGX0108 Cm+ could be detected while thyA+ *S. mutans* Clarke 1924 AL pILPOL Cm+ were still present at d3 and d7 **(****Figure 5****,** Panels C and D).
Relative to thyA+ *S. mutans* Clarke 1924 AL pILPOL Cm+, the presence of thyA- *S. mutans* sAGX0108 Cm+ showed an equally steep decrease in both cheek pouch as well as on dental surface (**Figure 5****,** Panels B and D).

The following Table 3 provides a summary of the strains used in the foregoing examples and their relevant characteristics (GUS: β-glucuronidase; Em: erythromycin; Cm chloramphenicol).

**Table 3**

| Strain | Source | relevant characteristics | | |
|---|---|---|---|---|
| | | GUS | Resistance to Em | Resistance to Cm |
| *S. mutans* strain Clarke 1924 AL | LMG | - | - | - |
| *S. mutans* strain Clarke 1924 AL pILPOL | AGX | - | + | - |
| *S. mutans* sAGX0108 | AGX | + | - | - |

The following Table 4 provides a summary of the abbreviations used here above.

| | |
|---|---|
| BHI | brain heart infusion broth |
| TF-BHI | thymidine free brain heart infusion broth |
| E | erythromycin |
| T | thymidine |
| X-Gluc | 5-bromo-4-chloro-3-indolyl-beta-D-glucuronic acid, cyclohexylammonium salt |
| GUS | β-glucuronidase |
| *thyA* | thymidylate synthase gene |
| uidA | E. coli β-glucuronidase gene |
| LMG | Laboratory of Microbiology Ghent |
| AGX | ActoGeniX |
| BAM9T | Carbonate and M9 salts buffer supplemented with amino acids and thymidine |
| BM9 | Carbonate and M9 salts buffer |

### References

Ajdic, D., W. M. McShan, et al. (2002). "Genome sequence of Streptococcus mutans UA159, a cariogenic dental pathogen." Proceedings of the National Academy of Sciences of the United States of America 99(22): 14434-9.
Allaker, R. P. and C. W. Douglas (2009). "Novel anti-microbial therapies for dental plaque-related diseases." International journal of antimicrobial agents 33(1): 8-13.
Altermann, E., W. M. Russell, et al. (2005). "Complete genome sequence of the probiotic lactic acid bacterium Lactobacillus acidophilus NCFM." Proceedings of the National Academy of Sciences of the United States of America 102(11): 3906-12.
Ausubel et al., Current Protocols in Molecular Biology, ed. Ausubel et al., Greene Publishing and Wiley-Interscience, New York, 1992 (with periodic updates)
Beighton, D. and H. Hayday (1982). "The streptococcal flora of the tongue of the monkey Macaca fascicularis." Archives of oral biology 27(4): 331-5.
Besier, S., A. Ludwig, et al. (2007). "Molecular analysis of the thymidine-auxotrophic small colony variant phenotype of Staphylococcus aureus." Int J Med Microbiol 297(4): 217-25.
Bigas, A., M. E. Garrido, et al. (2006). "Colonization capacity and serum bactericidal activity of Haemophilus parasuis thy mutants." Int Microbiol 9(4): 297-301.
Cazzaniga et al., Oral delayed release system for colonic specific delivery, Int. J. Pharm. 1994.
Chien, Novel drug delivery system, 2nd edition, M. Dekker, 1992
Claesson, M. J., Y. Li, et al. (2006). "Multireplicon genome architecture of Lactobacillus salivarius." Proceedings of the National Academy of Sciences of the United States of America 103(17): 6718-23.
Coykendall, A. L., D. Bratthall, et al. (1976). "Serological and genetic examination of some nontypical Streptococcus mutans strains." Infection and immunity 14(3): 667-70.
Davis, B. D. et al., Microbiology, 3rd edition, Harper & Row, publishers, Philadelphia, Pa., 1980.
Dent, V. E., J. M. Hardie, et al. (1978). "Streptococci isolated from dental plaque of animals." The Journal of applied bacteriology 44(2): 249-58.
Finegold, S. M., D. J. Flora, et al. (1975). "Fecal bacteriology of colonic polyp patients and control patients." Cancer research 35(11 Pt. 2): 3407-17.
Gehring, F., E. J. Karle, et al. (1976). "[The occurrence of Streptococcus mutans variants in man and laboratory animals]." Deutsche zahnarztliche Zeitschrift 31(1): 18-21.
Hamada, S., N. Masuda, et al. (1980). "Isolation and serotyping of Streptococcus mutans from teeth and feces of children." Journal of clinical microbiology 11(4): 314-8.
Hamada, S. and H. D. Slade (1980). "Biology, immunology, and cariogenicity of Streptococcus mutans." Microbiological reviews 44(2): 331-84.
Hamady, Z. Z., N. Scott, et al. (2009). "Xylan regulated delivery of human keratinocyte growth factor-2 to the inflamed colon by the human anaerobic commensal bacterium Bacteroides ovatus." Gut (ePub ahead of print).
Hansel et al., Pharmaceutical dosage forms and drug delivery systems, 5th edition, William and Wilkins, 1990.
Hasselbring, B. M., C. A. Page, et al. (2006). "Transposon mutagenesis identifies genes associated with Mycoplasma pneumoniae gliding motility." J Bacteriol 188(17): 6335-45.
Huber, G., J. Van Houte, et al. (1977). "Relationship between the populations of Streptococcus mutans in the mouth and feces of conventional Sprague-Dawley rats." Journal of dental research 56(12): 1614-9.
Innis et al., PCR Protocols: A Guide to Methods and Applications, Academic Press: San Diego, 1990.
Kilian, M., E. Theilade, et al. (1971). "Isolation of Streptococcus mutans from human faeces." Archives of oral biology 16(5): 553-4.
Kleerebezem, M., J. Boekhorst, et al. (2003). "Complete genome sequence of Lactobacillus plantarum WCFS1." Proceedings of the National Academy of Sciences of the United States of America 100(4): 1990-5.
Law, J., G. Buist, et al. (1995). "A system to generate chromosomal mutations in Lactococcus lactis which allows fast analysis of targeted genes." J Bacteriol 177(24): 7011-8.
Lehner, T., S. J. Challacombe, et al. (1975). "Immunological and bacteriological basis for vaccination against dental caries in rhesus monkeys." Nature 254(5500): 517-20.
Liljemark, W. F., S. V. Schauer, et al. (1978). "Compounds which affect the adherence of Streptococcus sanguis and Streptococcus mutans to hydroxyapatite." Journal of dental research 57(2): 373-9.
Maruyama, F., M. Kobata, et al. (2009). "Comparative genomic analyses of Streptococcus mutans provide insights into chromosomal shuffling and species-specific content." BMC genomics 10: 358.
Perez-Martinez, G., J. Kok, et al. (1992). "Protein export elements from Lactococcus lactis." Mol Gen Genet 234(3): 401-11.
Prescott et al., Novel drug delivery, J. Wiley & Sons, 1998
Proctor, R. A., P. van Langevelde, et al. (1995). "Persistent and relapsing infections associated with small-colony variants of Staphylococcus aureus." Clin Infect Dis 20(1): 95-102.
Sambrook et al., Molecular Cloning: A Laboratory Manual, 2nd ed., vol. 1-3, ed. Cold Spring Harbor Laboratory Press, Cold Spring Harbor, N.Y., 1989
Samuel, J. L. (1982). "The normal flora of the mouths of macropods (Marsupialia: macropodidae)." Archives of oral biology 27(2): 141-6.
Sasaki, Y., Y. Ito, et al. (2004). "ThyA as a selection marker in construction of food-grade host-vector and integration systems for Streptococcus thermophilus." Appl Environ Microbiol 70(3): 1858-64.
Sibakov, M., T. Koivula, et al. (1991). "Secretion of TEM beta-lactamase with signal sequences isolated from the chromosome of Lactococcus lactis subsp. lactis." Appl Environ Microbiol 57(2): 341-8.
Spanu, T., L. Romano, et al. (2005). "Recurrent ventriculoperitoneal shunt infection caused by small-colony variants of Staphylococcus aureus." Clin Infect Dis 41(5): e48-52.
Steidler, L., W. Hans, et al. (2000). "Treatment of murine colitis by Lactococcus lactis secreting interleukin-10." Science 289: 1352-5.
Steidler, L., S. Neirynck, et al. (2003). "Biological containment of genetically modified Lactococcus lactis for intestinal delivery of human interleukin 10." Nat. Biotechnol. 21: 785-9.
Steidler, L., K. Robinson, et al. (1998). "Mucosal delivery of murine interleukin-2 (IL-2) and IL-6 by recombinant strains of Lactococcus lactis coexpressing antigen and cytokine." Infect Immun 66(7): 3183-9.
Thomson, L. A., W. H. Bowen, et al. (1979). "Simultaneous implantation of five serotypes of Streptococcus mutans in gnotobiotic rats." Caries research 13(1): 9-17.
Unsworth, P. F. (1980). "The isolation of streptococci from human faeces." The Journal of hygiene 85(1): 153-64.
Wunder, J. A., W. W. Briner, et al. (1976). "Identification of the cultivable bacteria in dental plaque from the beagle dog." Journal of dental research 55(6): 1097-102.

In view of the foregoing, it will be appreciated that the invention described herein inter alia relates to the following items:
1. A method for reducing or abolishing the colonization capacity of a microbe, comprising rendering defective a thymidylate synthase (thyA) gene in said microbe.
2. The method according to item 1, wherein the rendering defective of the thyA gene results in thymidylate synthase deficiency in said microbe strain.
3. The method according to any one of items 1 or 2, wherein the microbe elicits a prophylactic and/or therapeutic effect in a subject, preferably in a human or animal.
4. The method according to any one of items 1 to 3, wherein the microbe expresses a heterologous expression product capable of eliciting a prophylactic and/or therapeutic response in a subject, preferably in a human or animal.
5. The method according to item 4, wherein said heterologous expression product is an antigen or a non-vaccinogenic prophylactically and/or therapeutically active peptide, polypeptide or protein.
6. The method according to any one of items 1 to 5 above, wherein the microbe is a bacterium, yeast or fungus.
7. The method according to any one of items 1 to 6 above, wherein the microbe is capable of residing in a mucosa, preferably in human or animal mucosa, more preferably in the mucosa of the alimentary tract.
8. The method according to any one of items 1 to 7, wherein the microbe is any of the species *Candida sp., Aspergillus sp., Penicillium sp., Saccharomyces sp., Hansenula sp., Kluyveromyces sp. Schizzosaccharomyces sp. Zygosaccharomyces sp., Pichia sp., Monascus sp., Geotrichum sp, Yarrowia sp. Bacteroides sp, Clostridium sp., Fusobacterium sp., Eubacterium sp., Ruminococcus sp., Peptococcus sp., Peptostreptococcus sp., Streptococcus sp., Bifidobacterium sp., Escherichia sp.* and *Lactobacillus sp.,* more preferably wherein the microbe is a *Bifidobacterium sp.* or a *Lactobacillus sp,* even more preferably wherein the microbe is a *Lactobacillus sp* chosen from a *Lactobacillus casei, Lactobacillus plantarum, Lactobacillus salivarius* or *Lactobacillus rhamnosus,* and any subspecies and strains thereof or wherein the microbe is a *Streptococcus sp* more preferably *Streptococcus mutans* and any subspecies and strains thereof, or wherein the microbe is a *Bacteroides sp. more preferably Bacteroides ovatus* and any subspecies and strains thereof.
9. Use of thymidylate synthase deficiency in a microbe for reducing or abolishing the colonization capacity of said microbe.
10. A microbe comprising an inactive thymidylate synthase gene (thyA), wherein said microbe has a reduced capacity of colonizing a mucosa of a human or animal in comparison to its wild type ancestor.
11. The microbe according to item 10, which is thymidylate synthase deficient.
12. A microbe according to any one of items 10 or 11, wherein the said microbe elicits a prophylactic and/or therapeutic effect in a subject, preferably in a human or animal.
13. A microbe according to item 10 to 12, wherein the said microbe further comprises a recombinant nucleic acid encoding a polypeptide capable of eliciting a therapeutic or immunogenic response in a subject, preferably in a human or animal.
14. A microbe according to item 13, wherein the said recombinant nucleic acid encodes an antigen and/or a non-vaccinogenic therapeutically active polypeptide.
15. A microbe according to item 14, wherein the said antigen is capable of eliciting an immune response, preferably an immune tolerance response, in a human or animal subject, and/or the said non-vaccinogenic therapeutically active polypeptide is capable of producing a therapeutic effect in a human or animal.
16. A microbe of any of the items 10 to 15, wherein the microbe is a bacterium, yeast or fungus.
17. A microbe of any of the items 10 to 16, wherein the microbe is capable of residing in a mucosa, preferably in mucosa of an animal or human subject, in particular in the mucosa of the alimentary tract.
18. A microbe of any of the items 10 to 17, wherein the microbe is any of the species *Candida sp., Aspergillus sp., Penicillium sp., Saccharomyces sp., Hansenula sp., Kluyveromyces sp. Schizzosaccharomyces sp. Zygosaccharomyces sp., Pichia sp., Monascus sp., Geotrichum sp, Yarrowia sp. Bacteroides sp, Clostridium sp., Fusobacterium sp., Eubacterium sp., Ruminococcus sp., Peptococcus sp., Peptostreptococcus sp., Streptococcus sp., Bifidobacterium sp.. Escherichia sp.* and *Lactobacillus sp.,* more preferably.wherein the microbe is a *Bifidobacterium sp.* or a *Lactobacillus sp,* even more preferably wherein the microbe is a *Lactobacillus sp.* chosen from a *Lactobacillus casei, Lactobacillus plantarum, Lactobacillus salivarius* or *Lactobacillus rhamnosus,* and any subspecies and strains thereof or wherein the microbe is a *Streptococcus sp* more preferably *Streptococcus mutans* and any subspecies and strains thereof, or wherein the microbe is a *Bacteroides sp. more preferably Bacteroides ovatus* and any subspecies and strains thereof.
19. A microbe according to any of items 10 to 18, as a reduced colonizing or a noncolonizing strain, for use as a medicament.
20. Use of a microbe according to any of items 10 to 18 as a reduced colonizing or a noncolonizing strain for the delivery of prophylactic and/or therapeutic molecules, preferably for the delivery of one or more heterologous expression products.

Additionally, the invention described herein also relates to the following items:
1. A microbe selected from the group consisting of a non-pathogenic, non-invasive Gram-positive bacterium, a yeast or a fungi comprising:
   (a) a defective thymidylate synthase *(thyA)* gene;
   (b) a recombinant nucleic acid operably linked to one or more open reading frames encoding one or more heterologous expression products which are capable of eliciting a therapeutic or immunologic response in said human or said animal,
   wherein said microbe has a reduced capacity of colonizing a mucosa of a human or animal, in comparison to its wild type ancestor, and
   wherein the recombinant nucleic acid comprises a native promoter from *Lactobacillus, Streptococcus, Bacteroides,* a functional variant or functional fragments thereof, or a native promoter of a *Lactococcus* gene selected from:
   (i) DNA-directed RNA polymerase, beta subunit / 160 kD subunit (*rpoC*);
   (ii) DNA-directed RNA polymerase, beta subunit / 140 kD subunit (*rpoB*);
   (iii) non-heme iron-binding ferritin (*dpsA*);
   (iv) pyruvate kinase (*pyk*);
   (v) glutamyl-tRNA synthetases (*gltX*);
   (vi) phosphopyruvate hydratase (eno);
   (vii) glutamine synthetase (*glnA*);
   (viii) glutamine synthetase repressor (*glnR*);
   (ix) dipeptidase PepV (pepV);
   (x) F0F1-type ATP synthase beta subunit (ATP synthase F1 beta subunit) (*atpD*);
   (xi) 3-phosphoglycerate kinase (*pgk*);
   (xii) glyceraldehyde-3-phosphate dehydrogenase (*gapB*);
   (xiii) acetate kinase (*ackA*);
   (xiv) 3-oxoacyl-(acyl-carrier-protein) synthase II (*fabF*);
   (xv) 3-ketoacyl- (acyl-carrier-protein) reductase (*fabG* or *fabG1*);
   (xvi) DNA-directed RNA polymerase, alpha subunit / 40 kD subunit (*rpoA*);
   (xvii) proline dipeptidase (pepQ);
   (xviii) fructose-bisphosphate aldolase (*fbaA*);
   (xix) ribosomal protein S4 (*rpsD*);
   (xx) superoxide dismutase (sodA);
   (xxi) ribosomal protein S12 (*rpsL*) and ribosomal protein S7 (*rpsG*);
   (xxii) ribosomal protein L18 (*rplR*) and ribosomal protein S5 (*rpsE*) and ribosomal protein L30/L7E (*rpmD*);
   (xxiii) S- ribosylhomocysteinase (*luxS*);
   (xxiv) ribosomal protein L19 (*rplS*);
   (xxv) ribosomal protein S11 (*rpsK* or *infA*);
   (xxvi) ribosomal protein L10 (*rplJ*);
   (xxvii) ribosomal protein L7/L12 (*rpIL);*
   (xxviii) HU-like DNA-binding protein (*hllA*);
   (xxix) 5OS ribosomal protein L28 (*rpmB*);
   (xxx) phosphotransferase system MB component (*ptcB*);
   (xxxi) F0F1-type ATP synthase alpha subunit (*atpA*);
   (xxxii) multiple sugar-binding transport ATP-binding protein (*msmK*);
   (xxxiii) pyruvate dehydrogenase E1 component alpha subunit (*pdhA*);
   (xxxiv) cell division protein (*diflVA* or *ftsA*);
   (xxxv) UDP-galactopyranose mutase (*glf1*);
   (xxxvi) glutamyl aminopeptidase (*pepA*);
   (xxxvii) predicted dehydrogenase related protein (ll*mg_0272*);
   (xxxviii) ribosomal protein S2 (*rpsB*);
   (xxxix) translation initiation factor 3 (IF-3) (*infC*);
   (xl) ribosomal protein L4 (*rpID*) and ribosomal protein L23 (*rplW*) and ribosomal protein L2 (*rplB*);
   (xli) phenylalanyl-tRNA synthetase beta chain (*pheT*);
   (xlii) transcription elongation factor GreA (*greA*);
   (xliii) ATP-dependent Clp protease proteolytic subunit (*clpP*);
   (xliv) ribosomal protein L15 (*rplO*);
   (xlv) ribosomal protein L11 (*rplK*);
   (xlvi) ribosomal protein S8 (*rpsH*);
   (xlvii) ribosomal protein L21 (*rplU*);
   (xlviii) ribosomal protein S13 (*rpsM*);
   (xix) ribosomal protein S19 (*rpsS*) and ribosomal protein L22 (*rplV*) and ribosomal protein L16 (*rplP*) and ribosomal protein L14 (*rplN*);
   (l) ribosomal protein S10 (*rpsJ*);
   (li) co-chaperonin GroES (Hsp10) (*groES*);
   (lii) ribosomal protein L24 (*rpIX*);
   (liii) putative holiday junction resolvase *(llmg_0151*); and
   (liv) functional variants and functional fragments thereof.
2. The microbe according to item 1, wherein the said recombinant nucleic acid encoding the one or more heterologous expression products is comprised in a vector.
3. The microbe according to item 1 or item 2, wherein the said one or more heterologous expression products is a peptide, polypeptide or protein, preferably an antigen and/or non-vaccinogenic prophylactically and/or therapeutically active peptide, polypeptide or protein.
4. The microbe according to item 3, wherein the said antigen is capable of eliciting an immune tolerance response in the said human or said animal.
5. The microbe according to any one of items 1 to 4, wherein the said one or more heterologous expression products is selected from: insulin, a growth hormone, prolactin, calcitonin, luteinising hormone, parathyroid hormone, somatostatin, thyroid stimulating hormone, vasoactive intestinal polypeptide, a cytokine, an insulin related cytokine, a receptor or antagonist thereof, hIL-10, GLP-2, GLP-1, TFF or hPYY.
6. A method for reducing or abolishing the colonization capacity of a microbe, wherein said microbe is capable of residing in the mucosa of a human or animal and wherein said microbe comprises a recombinant nucleic acid which encodes one or more heterologous expression products capable of eliciting a prophylactic and/or therapeutic response in said human or animal, wherein said method comprises rendering defective a thymidylate synthase *(thyA)* gene in said microbe, and
   wherein said one or more heterologous expression products is selected from: insulin, a growth hormone, prolactin, calcitonin, luteinising hormone, parathyroid hormone, somatostatin, thyroid stimulating hormone, vasoactive intestinal polypeptide, a cytokine, an insulin related cytokine, a receptor or antagonist thereof, hIL-10, GLP-2, GLP-1, TFF or hPYY.
7. The microbe according to any one of items 1 to 5 or the method according to item 6, wherein said defective *thyA* gene is an endogenous *thyA* gene, preferably wherein the endogenous *thyA* gene is chromosomally located.
8. The microbe or method according to any one of items 1 to 7, wherein the defective *thyA* gene is a recombinant defective *thyA* gene, preferably wherein the *thyA* gene is situated in the chromosome and is inactivated by gene disruption.
9. The microbe or method according to any one of items 1 to 8, wherein the microbe is thymidylate synthase deficient.
10. The microbe or method according to any one of items 1 to 9, wherein said microbe elicits a prophylactic and/or therapeutic effect in said human or said animal.
11. The microbe or method according to any one of items 1 to 10, wherein the microbe further comprises suitable regulatory sequences operably linked to one or more open reading frames (ORFs) encoding the said one or more heterologous expression products, preferably wherein the microbe further comprises a sequence encoding a secretion signal in phase with a polypeptide encoded by the said one or more ORFs.
12. The microbe or method according to any one of items 5 to 11, wherein the cytokine is selected from: an interleukin, GM-CSF, M-CSF, SCF, IFNs, EPO, G-CSF, LIF, OSM, CNTF, GH, PRL, a TNF family member, TNFα, TNPβ, CD40, CD27, a FAS ligand, an IL-1 family member, a fibroblast growth factor family member, a platelet-derived growth factor, a transforming growth factor, a nerve growth factor, and an epidermal growth factor family member.
13. The microbe or method according to item 12, wherein the interleukin is selected from: IL-2, IL-3, IL-4, IL-5, IL-6, IL-7, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-34 and IL-35.
14. The microbe or method according to any one of items 1 to 13, wherein the promoter is a native promoter of a *Lactococcus* gene selected from:
   (a) phosphopyruvate hydratase (*eno*);
   (b) glyceraldehyde-3-phosphate dehydrogenase (*gapB*); and
   (c) HU-like DNA-binding protein (*hllA*);
15. The microbe or method according to any one of items 1 to 13, wherein the promoter is a native promoter of a *Lactobacillus* gene selected from:
   (a) ribosomal protein S14 and ribosomal protein S17 and ribosomal protein L15 and ribosomal protein S3 (*rpsJ*);
   (b) nucleoid DNA-binding protein (*dnabp*);
   (c) ribosomal protein S21 (*rpS21*);
   (d) 50S ribosomal protein L19 (*rplS*);
   (e) 50S ribosomal protein L17 (*rnap40*);
   (f) 50S ribosomal protein L13 (*rplM*);
   (g) phosphoglycerate mutase 1 (*pgm1*);
   (h) ribosomal protein S4 (*rpS4*);
   (i) glyceraldehyde-3-phosphate dehydrogenase (*cggr*); and
   (j) functional variants and functional fragments thereof.
16. The method according to any of the items 6 to 15, wherein the microbe is a bacterium, yeast or fungus, preferably wherein the said yeast or fungi is selected from the group consisting of *Candida* sp., *Aspergillus* sp., *Penicillium* sp., *Saccharomyces* sp., *Hansenula* sp., *Kluyveromyces* sp., *Schizzosaccharomyces* sp., *Zygosaccharomyces* sp., *Pichia* sp., *Monascus* sp., *Geotrichum* sp. and *Yarrowia* sp.
17. The method according to item 16, wherein the said bacterium is a non-pathogenic, non-invasive Gram-positive bacterium.
18. The method according to item 16, wherein the said bacterium is selected from the group consisting of *Bacteroides* sp., *Clostridium* sp., *Fusobacterium* sp., *Eubacterium* sp., *Ruminococcus* sp., *Peptococcus* sp., *Peptostreptococcus* sp., *Streptococcus* sp., *Bifidobacterium* sp., *Escherichia* sp. *Lactobacillus* sp., and any subspecies or strains thereof.
19. The microbe according to any one of items 1 to 5 or 7 to 15, wherein the said bacterium is selected from the group consisting of *Clostridium* sp., *Eubacterium* sp., *Ruminococcus* sp., *Peptococcus* sp., *Peptostreptococcus* sp., *Streptococcus* sp., *Bifidobacterium* sp., *Lactobacillus* sp., and any subspecies or strains thereof.
20. A pharmaceutical composition comprising the microbe according to any one of items 1 to 5 or 7 to 15 and a pharmaceutically acceptable carrier.
21. A starter culture, inoculum, lyophilized composition, frozen liquid composition, or food and feed composition comprising the microbe according to any one of items 1 to 5 or 7 to 15.
22. The microbe according to any one of items 1 to 5 or 7 to 15 for use in treating a disease selected from the group consisting of: an inflammatory bowel disease, an autoimmune disease, a neurological disorder, cancer, osteoporosis, diabetes, cardiovascular disease, atherosclerosis, hemophilia, degenerative liver disease, a pulmonary disease, obesity, and pathogen infections.
23. The microbe according to any one of items 1 to 5 or 7 to 15, wherein the said bacterium is selected from the group consisting of *Clostridium* sp., *Eubacterium* sp., *Ruminococcus* sp., *Peptococcus* sp., *Peptostreptococcus* sp., *Streptococcus* sp., *Bifidobacterium* sp., and any subspecies or strains thereof.

## Claims

1. A non-pathogenic, non-invasive Gram-positive bacterium comprising:
(a) a defective thymidylate synthase *(thyA)* gene;
(b) a recombinant nucleic acid operably linked to one or more open reading frames encoding one or more heterologous expression products which are capable of eliciting a therapeutic or immunologic response in said human or said animal,
or a pharmaceutical composition comprising said bacterium, for use in treating oral mucositis, an autoimmune disease, a neurological disorder, cancer, osteoporosis, diabetes, cardiovascular disease, atherosclerosis, hemophilia, degenerative liver disease, a pulmonary disease, obesity, or pathogen infections, wherein said bacterium has a reduced capacity of colonizing a mucosa of a human or animal, in comparison to its wild type ancestor, and wherein the bacterium is to be administered to the animal or human by the oral mucosa.

2. The non-pathogenic, non-invasive Gram-positive bacterium, or pharmaceutical composition for the use according to claim 1, wherein the bacterium is for use in treating oral mucositis.

3. The non-pathogenic, non-invasive Gram-positive bacterium, or pharmaceutical composition for the use according to claim 1 or claim 2, wherein the said recombinant nucleic acid encoding the one or more heterologous expression products is comprised in a vector.

4. The non-pathogenic, non-invasive Gram-positive bacterium, or pharmaceutical composition for the use according to any one of claims 1 to 3, wherein the said one or more heterologous expression products is a peptide, polypeptide or protein, preferably an antigen and/or non-vaccinogenic prophylactically and/or therapeutically active peptide, polypeptide or protein.

5. The non-pathogenic, non-invasive Gram-positive bacterium, or pharmaceutical composition for the use according to claim 4, wherein the said antigen is capable of eliciting an immune tolerance response in the said human or said animal.

6. The non-pathogenic, non-invasive Gram-positive bacterium, or pharmaceutical composition for the use according to any one of claims 1 to 5, wherein the said one or more heterologous expression products is selected from: insulin, a growth hormone, prolactin, calcitonin, luteinising hormone, parathyroid hormone, somatostatin, thyroid stimulating hormone, vasoactive intestinal polypeptide, a cytokine, an insulin related cytokine, a receptor or antagonist thereof, hIL-10, GLP-2, GLP-1, TFF or hPYY.

7. The non-pathogenic, non-invasive Gram-positive bacterium, or pharmaceutical composition for the use according claim 6, wherein the cytokine is selected from: an interleukin, GM-CSF, M-CSF, SCF, IFNs, EPO, G-CSF, LIF, OSM, CNTF, GH, PRL, a TNF family member, TNFa, TNFβ, CD40, CD27, a FAS ligand, an IL-1 family member, a fibroblast growth factor family member, a platelet-derived growth factor, a transforming growth factor, a nerve growth factor, and an epidermal growth factor family member.

8. The non-pathogenic, non-invasive Gram-positive bacterium, or pharmaceutical composition for the use according to claim 7, wherein the interleukin is: IL-2, IL-3, IL-4, IL-5, IL-6, IL-7, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-34 or IL-35.

9. The non-pathogenic, non-invasive Gram-positive bacterium, or pharmaceutical composition for the use according to any one of claims 1 to 8, wherein the said bacterium is a *Bacteroides* sp., *Clostridium* sp., *Fusobacterium* sp., *Eubacterium* sp., *Ruminococcus* sp., *Peptococcus* sp., *Peptostreptococcus* sp., *Streptococcus* sp., *Bifidobacterium* sp., *Escherichia* sp. *Lactobacillus* sp., or any subspecies or strains thereof.

10. The non-pathogenic, non-invasive Gram-positive bacterium, or pharmaceutical composition for the use according to claim 9, wherein the said bacterium is a *Streptococcus* sp., preferably S. *mutans.*

11. The non-pathogenic, non-invasive Gram-positive bacterium, or pharmaceutical composition for the use according to any one of claims 1 to 10, wherein said defective *thyA* gene is an endogenous *thyA* gene, preferably wherein the endogenous *thyA* gene is chromosomally located.

12. The non-pathogenic, non-invasive Gram-positive bacterium, or pharmaceutical composition for the use according to any one of claims 1 to 11, wherein the defective *thyA* gene is a recombinant defective *thyA* gene, preferably wherein the *thyA* gene is situated in the chromosome and is inactivated by gene disruption.

13. The non-pathogenic, non-invasive Gram-positive bacterium, or pharmaceutical composition for the use according to any one of claims 1 to 12, wherein the bacterium further comprises suitable regulatory sequences operably linked to one or more open reading frames (ORFs) encoding the said one or more heterologous expression products, preferably wherein the microbe further comprises a sequence encoding a secretion signal in phase with a polypeptide encoded by the said one or more ORFs.

14. The non-pathogenic, non-invasive Gram-positive bacterium, or pharmaceutical composition for the use according to any one of claims 1 to 13, wherein the recombinant nucleic acid comprises a native promoter of a *Lactococcus* gene selected from the group consisting of: glyceraldehyde-3-phosphate dehydrogenase (*gapB*)*,* non-heme iron-binding ferritin (*dpsA*)*,* pyruvate kinase (*pyk*)*,* phosphopyruvate hydratase (eno), dipeptidase PepV (*pepV*)*,* superoxide dismutase (*sodA*), ribosomal protein L19 (*rplS*), HU-like DNA binding protein (*hll*A*),* and 50S ribosomal protein L28 (*rpmB*).

15. The non-pathogenic, non-invasive Gram-positive bacterium, or pharmaceutical composition for the use according to any one of claims 1 to 14, wherein the recombinant nucleic acid comprises:
(a) *PdpsA,* usp45 and hTFF1;
(b) *PdpsA,* usp45 and hTFF1;
(c) *Ppepv,* usp45 and hTFF1;
(d) *Ppepv,* usp45 and hTFF1;
(e) *PsodA,* usp45 and hTFF1;
(f) *PsodA,* usp45 and hTFF1;
(g) *PhllA,* usp45 and hTFF1;
(h) *PhllA,* usp45 and hTFF1;
(i) *PdpsA,* usp45 and hTFF3;
(j) *PdpsA,* usp45 and hTFF3;
(k) *Ppepv,* usp45 and hTFF3;
(l) *Ppepv,* usp45 and hTFF3;
(m) *PsodA,* usp45 and hTFF3;
(n) *PsodA,* usp45 and hTFF3;
(o) *PhllA,* usp45 and hTFF3; or
(p) *PhllA,* usp45 and hTFF3.

16. The pharmaceutical composition for the use according to any one of claims 1 to 15, further comprising a pharmaceutically acceptable carrier.

17. A non-pathogenic, non-invasive Gram-positive bacterium comprising:
(a) a defective thymidylate synthase *(thyA)* gene;
(b) a recombinant nucleic acid operably linked to one or more open reading frames encoding one or more heterologous expression products which are capable of eliciting a therapeutic or immunologic response in said human or said animal,
wherein said bacterium has a reduced capacity of colonizing the oral mucosa of a human or animal, in comparison to its wild type ancestor.
